(19) Europäisches Patentamt
European Patent Office
Office européen des brevets

(11) EP 3 969 063 B1

(12) **EUROPEAN PATENT SPECIFICATION**

(45) Date of publication and mention of the grant of the patent:
**05.07.2023 Bulletin 2023/27**

(21) Application number: **20723888.2**

(22) Date of filing: **12.05.2020**

(51) International Patent Classification (IPC):
***A61K 49/00*** (2006.01) ***C07D 417/14*** (2006.01)
***C07D 403/12*** (2006.01) ***C07D 209/14*** (2006.01)
***C07K 16/28*** (2006.01) ***C07K 7/52*** (2006.01)
***A61K 39/00*** (2006.01) ***C07D 471/18*** (2006.01)
***C09B 23/06*** (2006.01) ***C09B 23/08*** (2006.01)

(52) Cooperative Patent Classification (CPC):
**A61K 49/0032; A61K 49/0052; A61K 49/0056; A61K 49/0058; C07D 209/14; C07D 403/12; C07D 417/14; C07D 471/18; C07K 7/52; C07K 16/2863; C09B 23/06; C09B 23/083; C09B 23/086;** A61K 2039/505; C07K 2317/21;

(Cont.)

(86) International application number:
**PCT/EP2020/063114**

(87) International publication number:
**WO 2020/229438 (19.11.2020 Gazette 2020/47)**

(54) **MODIFIED CYANINE DYES AND CONJUGATES THEREOF**

MODIFIZIERTE CYANINFARBSTOFFE UND KONJUGATE DAVON

COLORANTS DE CYANINE MODIFIÉS ET LEURS CONJUGUÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.05.2019 EP 19174082**

(43) Date of publication of application:
**23.03.2022 Bulletin 2022/12**

(60) Divisional application:
**23168594.2**

(73) Proprietor: **Bracco Imaging SPA**
**20134 Milan (IT)**

(72) Inventors:
- **BLASI, Francesco**
  **10010 Colleretto Giacosa (IT)**
- **BRIOSCHI, Chiara**
  **10010 Colleretto Giacosa (IT)**
- **BUONSANTI, Federica**
  **10010 Colleretto Giacosa (IT)**
- **MIRAGOLI, Luigi**
  **10010 Colleretto Giacosa (IT)**
- **NAPOLITANO, Roberta**
  **10010 Colleretto Giacosa (IT)**
- **ORIO, Laura**
  **10010 Colleretto Giacosa (IT)**
- **PIZZUTO, Lorena**
  **10010 Colleretto Giacosa (IT)**
- **VALBUSA, Giovanni**
  **28838 Stresa (IT)**

(74) Representative: **Ravizza, Claudio**
**Bracco Imaging SpA**
**Intellectual Property**
**Via Caduti di Marcinelle, 13**
**20134 Milano (IT)**

(56) References cited:
**WO-A1-2018/189136**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **CAPOZZA M ET AL: "Photoacoustic imaging of integrin-overexpressing tumors using a novel ICG-based contrast agent in mice", PHOTOACOUSTICS, vol. 11, 1 September 2018 (2018-09-01), pages 36-45, XP055634719, ISSN: 2213-5979, DOI: 10.1016/j.pacs.2018.07.007**
- **EBERT B ET AL: "Cyanine dyes as contrast agents for near-infrared imaging in vivo: acute tolerance, pharmacokinetics, and fluorescence imaging", JOURNAL OF BIOMEDICAL OPTICS, vol. 16, no. 6, 1 January 2011 (2011-01-01), page 066003, XP055634009, ISSN: 1083-3668, DOI: 10.1117/1.3585678**



(52) Cooperative Patent Classification (CPC): (Cont.)
C07K 2317/24; C07K 2317/92; C07K 2317/94

## Description

### FIELD OF THE INVENTION

The present invention relates to the field of optical imaging. More particularly, it relates to compounds of the cyanine family characterized by improved physico-chemical and biological properties and to their conjugates with biological ligands thereof. The invention also relates to the use of these compounds as optical diagnostic agents in imaging or therapy of solid tumors, to the methods for their preparation and to the compositions comprising them.

### BACKGROUND ART

Dyes are chemical entities that absorb photons of a specific wavelength upon light excitation and re-emit some of that energy, depending on quantum efficiency, usually at a longer wavelength. Particularly, cyanine dyes are fluorescent organic molecules characterized by a delocalized electron system that spans over a polymethine bridge and is confined between two nitrogen atoms. Some of them, having favourable optical properties, low toxicity and good solubility in aqueous media, can be used as contrast agents for biomedical imaging.

Fluorescent molecules currently used for biomedical imaging, such as Indocyanine green (ICG), Fluorescein, Methylene Blue and 5-Aminolevulenic acid (5-ALA), passively diffuse in tissues and may accumulate in pathological regions thanks to different homing mechanisms. Fluorophores like Fluorescein and ICG distribute in tumor tissues by a combination of passive diffusion and enhanced permeability and retention (EPR) effect (Onda N. et al., Int J Cancer 2016, 139, 673-682). Metabolic intermediates like 5-ALA accumulate in tissues with increased metabolism (Stummer W. et al., Neurosurgery 2015, 77(5), 663-673). Other fluorophores, not directed against a specific molecular target, take advantage of tumor-physiological properties (i.e., enhanced perfusion and permeability) to generate the image contrast, following the principles of contrast-enhanced X-ray or magnetic resonance imaging (Licha et al., Photochemistry and Photobiology, 2000, 72(3), 392-398). In general, these fluorescent contrast agents require a relatively large mass dose for proper visualization. Moreover, false positive and false negative are common clinical findings associated with their use (Tummers Q. et al., PlosOne 2015).

Further contrast agents for optical imaging are under development which exploit the use of a dye conjugated to a carrier moiety, targeting an overexpressed tumor receptor, to improve sensitivity and specificity of detection (Achilefu S. et al, J Med Chem 2002, 45, 2003-2015).

However, the *in vivo* behavior of dye-biomolecule conjugates may be strongly affected by the biological properties of the fluorescent moiety. For example, small structural modifications of a cyanine Cy5 strongly modulate the accumulation in tumor and off-target tissues of the relative bioconjugates (Bunschoten A. et al., Bioconjugate Chem. 2016, 27, 1253-1258). Fluorescent contrast agents with low non-specific accumulation and high selectivity for the target tissue would be preferable for applications in living organisms.

US 6,913,743 B2 in the name of Institut fur Diagnostikforschung GmbH discloses for instance an *in vivo* diagnostic process using new water-soluble dyes and their biomolecule adducts as constrast medium.

WO2018/189136 and WO2016/097317, both in the name of Bracco Imaging SpA, disclose the compound DA364 used for the demarcation of tumor margins in intraoperative imaging. Such compound is an aza-bicycloalkane based cyclic RGD peptide conjugated with a sulfo-Cy5.5 dye.

An example of conjugate of the ICG, which is linked to a cRGD peptide, is disclosed in Capozza et al, Photoacoustics, 2018, 11, 36-45, where it is used for photoacoustic imaging of tumors.

US 6,329,531 B1, in the name of Schering AG, relates to optical diagnostic agents for diagnosis of neurodegenerative diseases by means of NIR radiation, and discloses in particular heptamethine cyanine dyes as fluorescent labels. Some of these cyanine dyes are also disclosed for instance in papers Licha et al., Photochemistry and Photobiology, 2000, 72(3), 392-398 and Ebert et al., J. Biomed. Optics, 2011, 16(6), 066003, where they are compared with the clinically approved dye ICG. In particular, the latter disclose a compound named SIDAG, which showed physico-chemical properties similar to those of MRI contrast media and, unlike ICG, was characterized by an extravascular distribution, with distribution volumes in the range of the extracellular water. SIDAG is a hydrophilic alternative to ICG designed to solve the technical problem of its negligible extravasation and rapid liver uptake. However, no mention was made about the possible use of such compounds, and in particular SIDAG, as fluorescent probes for molecular imaging and about any synthetic approach for a functional derivatization and further conjugation of such symmetric cyanine derivatives. For instance, SIDAG was proposed as biologically inert dye with extravascular distribution pattern to mimic the behavior of MRI contrast agents like Gadopentetic acid (Gd-DTPA). SIDAG can diffuse rapidly through pores of altered tumor vessels and can thus reach deeper regions of tumor tissue via passive diffusion, but it lacks the desired features for molecular imaging applications, such as the ability to internalize and accumulate with high efficiency and specificity within pathologic cells and tissues.

WO2005/123768, in the name of Amersham Health AS, describes new peptide-based compounds labelled

with at least one cyanine dye reporter suitable as contrast agent in optical imaging for diagnosis of angiogenesis related diseases.

WO2012/088007, in the name of Pierce Biotechnology Inc. and Dyomics Gmbh, discloses cyanine dyes having from two to four sulfonic acid functions on the indole heterocycle scaffold.

Despite several efforts to find suitable imaging agents, there is still the need to find improved dyes endowed with optimal stability and fluorescence efficiency, as well as optimal physicochemical and biological properties, and designed for optical imaging of living organisms. This need is paramount particularly for molecular imaging applications requiring highly specific tools, with ability to image fine molecular changes and to accumulate with high efficiency in target cells, such as when the dye is conjugated to a biomolecule that specifically binds a molecular epitope or a pathologic tissue (e.g. a tumor). The present invention addresses these and other needs.

## SUMMARY OF THE INVENTION

Generally, object of the present invention is to provide new cyanine dyes, and in particular their corresponding conjugates to binding moieties, useful as contrast medium for optical imaging and aimed at solving the above mentioned issues. Particularly, the present invention addresses the technical problem of obtaining an optical imaging agent with optimal properties for molecular imaging applications. For instance, the new cyanine derivatives of the invention have been found to display particularly suitable properties to interact with biological substrates and to accumulate in pathological cells and tissues.

The new cyanine derivatives described herein are surprisingly endowed with remarkable optical properties leading to an improved imaging efficiency at low mass doses and an improved signal-to-noise ratio. Furthermore, the present inventors have found suitable procedures to synthetize the new cyanine dyes and to conjugate them to different targeting moieties through suitable functional groups acting as binding sites, thus providing very specific and sensitive contrast agents for optical imaging. The novel combinations of dye, spacer and targeting moiety, as provided in the present invention, has surprisingly afforded compounds endowed with optimal cell internalization efficiency and the overall properties for molecular imaging to an optical imaging agent.

In detail, among the several advantages that can be achieved by means of the present compounds, particularly for the conjugates with a binding moiety, the following features can be highlighted for instance: low plasma proteins interaction, selectivity for the target tissue and low accumulation due to non-specific interaction with other tissues, high solubility in water, negligible or no observed adverse reactions after systemic administration, good chemical and optical stability in plasma after administration.

In particular, it has been found that the compounds of the invention have a very low binding affinity for human albumin, which is particularly advantageous when these compounds are used for imaging in humans: said low affinity prevents the sequestration of the compounds in the plasma compartments by the large proteins present in the blood, such as albumin, and the consequent reduction of the fraction of free dye available for accumulation in the tissue of interest. In fact, the albumin-bound fraction of dyes would accumulate in tissues at the transport rate of a macromolecule, since the high molecular weight of the albumin dictates the behaviour of the bound dye, and would cause their unwanted non-specific accumulation in healthy tissues and organs. This biological property is particularly important in case of dyes-conjugates, since only their free fraction (not bound to albumin) can properly interact with the molecular target and can efficiently be internalized by the cells expressing a particular antigen.

A further aspect of the invention relates to such dyes-conjugates as diagnostic agents, in particular for use in optical imaging of a human or animal organ or tissue, for use in a method of optical imaging, wherein the imaging is a tomographic imaging of organs, monitoring of organ functions including angiography, urinary tract imaging, bile duct imaging, nerve imaging, intraoperative cancer identification, fluorescence-guided surgery, fluorescence life-time imaging, short-wave infrared imaging, fluorescence endoscopy, fluorescence laparoscopy, robotic surgery, open field surgery, laser guided surgery, or a photoacoustic or sonofluorescence method.

Moreover the invention relates to a manufacturing process for the preparation of the provided dyes, the corresponding conjugates and/or the pharmaceutically acceptable salts thereof, and to their use in the preparation of a diagnostic agent.

According to a further aspect, the invention relates to a pharmaceutically acceptable composition comprising at least one dye or dye-conjugate compound of the invention, or a pharmaceutically acceptable salt thereof, in a mixture with one or more physiologically acceptable carriers or excipients. Said compositions are useful in particular as optical imaging agents to provide useful imaging of human or animal organs or tissues.

In another aspect, the present invention refers to a method for the optical imaging of a body organ, tissue or region by use of an optical imaging technique that comprises the use of an effective dose of a compound of the invention.

## DETAILED DESCRIPTION OF THE INVENTION

Accordingly, it is a first object of the present invention the provision of a compound of formula (I), or a pharmaceutically acceptable salt thereof,

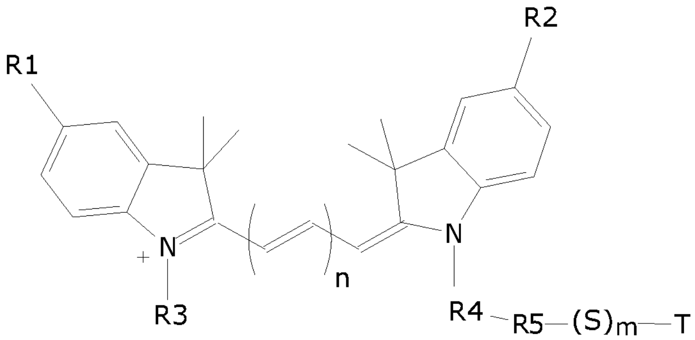

(I)

wherein

**R1** and **R2** are independently a -CO-NH-Y group, wherein Y is selected from a linear or branched alkyl, cycloalkyl and heterocyclyl, substituted by at least two hydroxyl groups;
**R3** is linear or branched alkyl substituted by a group selected from $-NH_2$, $-SO_3H$, -COOH and - $CONH_2$;
**R4** is linear or branched bivalent alkyl;
**R5** is selected from -COO- and -CONH-;
**S** is a spacer;
**T** is a targeting moiety;
**n** is an integer equal to 1, 2 or 3; and
**m** is an integer equal to 0 or 1.

The present invention further provides the corresponding functionalized dyes represented by an intermediate compound of formula (II), or a pharmaceutically acceptable salt thereof,

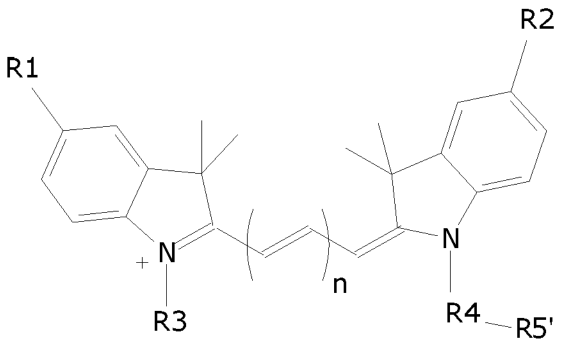

(II)

wherein

**R1, R2, R3, R4** and **n** are as defined above and
**R5'** is selected from -COOH, $-CONH_2$, -COO-Pg and -CONH-Pg, wherein Pg is a protecting group.

The present invention also relates to methods for preparing the compounds of formula (I) or (II) by means of synthetic transformations steps.

The invention also comprises compounds of formula (I) for use as fluorescent probes for the detection of a tumor margin in guided surgery.

*Definitions*

In the present description, and unless otherwise provided, the following terms and phrases as used herein are intended to have the following meanings.

The expression "straight or branched alkyl" refers to an aliphatic hydrocarbon radical group, which may be a straight or branched-chain, having from 1 to 8 carbon atoms in the chain. For instance, "$C_4$ alkyl" comprises within its meaning a linear or branched chain comprising 4 carbon atoms. Representative and preferred alkyl groups include methyl, ethyl, n-propyl, iso-propyl, n-butyl, tert-butyl, pentyl and hexyl. Unless otherwise specified, the straight or branched alkyl is a monovalent radical group. In some cases it may be a "bivalent" or "multivalent" radical group, wherein two or more hydrogen atoms are removed from the above hydrocarbon radical group and substituted, e.g. methylene, ethylene, iso-propylene groups and the like. The term "cycloalkyl" as used therein comprises within its meaning a saturated (i.e. cycloaliphatic) carbocyclic ring comprising from 3 to 7 carbon atoms. Suitable examples include a $C_5$-$C_7$ carbocyclic ring, e.g. a cyclohexyl ring.

The term "heterocyclyl" as used therein comprises a saturated cycloaliphatic ring, preferably a 5-7 membered saturated ring, further comprising an heteroatom in the cyclic chain selected from N, O and S. Preferably, it refers to tetrahydropyran.

The term "hydroxyalkyl" refers to any of the corresponding alkyl chain wherein one or more hydrogen atoms are replaced by hydroxyl groups.

The term "alkoxy" comprises within its meaning an alkyl chain as above defined further comprising one or more oxygen atoms; examples include, for instance, alkyl-oxy groups such as methoxy, ethoxy, n-propoxy, iso-propoxy and the like, and alkyl-(poly)oxy groups in which the alkyl chain is interrupted by one or more oxygen atoms.

In the present description the term "protecting group" (Pg) designates a protective group adapted for preserving the function of the group to which it is bound. Specifically, protective groups are used to preserve amino, hydroxyl or carboxyl functions. Appropriate protective groups may include, for example, benzyl, carbonyl, such as formyl, 9-fluoromethyloxycarbonyl (Fmoc), benzyloxycarbonyl (Cbz), t-butoxycarbonyl (Boc), isopropyloxycarbonyl or allyloxycarbonyl (Alloc), alkyl, e.g. tert-butyl or triphenylmethyl, sulfonyl, acetyl groups, such as trifluoroacetyl, benzyl esters, allyl, or other substituents commonly used for protection of such functions, which are well known to the person skilled in the art (see, for instance, the general reference T.W. Green and P.G.M.Wuts, Protective Groups in Organic Synthesis, Wiley, N.Y. 2007, 4th Ed., Ch. 5). More particularly, the invention comprises compounds of formula (II) in which the functional groups of R5', i.e. a carboxylic acid or carboxamide, are protected with an appropriate protecting group (Pg) as defined above, preferably with alkyl groups. Such derivatives find for instance application as suitable precursors or intermediate compounds in the preparation of a desired compound of formula (I) or salts thereof.

If necessary, hydroxyl groups of R1 and R2 and/or the functional groups of R3 can be also protected with an appropriate protecting group (Pg) during the preparation of the compounds of formula (I) or (II), thus forming for instance acetoxy, alkoxy, ester or amide groups.

The expression "coupling reagent" refers to a reagent used for instance in the formation of an amide bond between a carboxyl moiety and an amino moiety. The reaction may consist of two consecutive steps: activation of the carboxyl moiety and then acylation of the amino group with the activated carboxylic acid. Non limiting examples of such coupling agents are selected from the group consisting of: carbodiimides, such as N,N'-diisopropylcarbodiimide (DIC), N,N'-dicyclohexylcarbodiimide (DCC), 1-ethyl-3-(3-dimethylaminopropyl) carbodiimide (EDAC), 1-ethyl-3-(3-dimethylaminopropyl)carbodiimide (EDC) and 1-ethyl-3-(3-dimethylaminopropyl)carbodiimide (WSC); phosphonium reagents, such as (benzotriazol-1-yloxy)tris(dimethylamino)phosphonium hexafluorophosphate (BOP), (benzotriazol-1-yloxy)tripyrrolidinophosphonium hexafluorophosphate (PyBOP), 7-azabenzotriazol-1-yloxy-tripyrrolidino-phosphonium hexafluorophosphate (PyAOP), [ethyl cyano(hydroxyimino)acetato-O2]tri-1-pyrrolidinylphosphonium hexafluorophosphate (PyOxim), bromotripyrrolidinophosphonium hexafluorophosphate (PyBrOP) and 3-(diethoxyphosphoryloxy)-1,2,3-benzotriazin-4(3H)-one (DEPBT); and aminium/uronium-imonium reagents, such as O-(N-Succinimidyl)-1,1,3,3-tetramethyluronium tetrafluoroborate (TSTU), N,N,N',N'-tetramethyl-O-(benzotriazol-1-yl)uronium tetrafluoroborate (TBTU), N,N,N',N'-tetramethyl-O-(1H-benzotriazol-1-yl)uronium hexafluorophosphate (HBTU), N,N,N',N'-tetramethyl-O-(7-azabenzotriazol-1-yl)uronium hexafluorophosphate (HATU), O-(1H-6-chlorobenzotriazole-1-yl)-1,1,3,3-tetramethyluronium hexafluorophosphate (HCTU), 1-[1-(cyano-2-ethoxy-2-oxoethylidene-aminooxy)-dimethylamino-morpholino]-uronium hexafluorophosphate (COMU) and fluoro-N,N,N',N'-tetramethylformamidinium hexafluorophosphate (TFFH) or other compounds well known to the person skilled in the art.

The expression "activated carboxylic acid" refers to a derivative of a carboxyl group that is more susceptible to nucleophilic attack than a free carboxyl group; suitable derivatives may include for instance acid anhydrides, thioesters, acyl halides, NHS ester and sulfo NHS esters.

The terms "moiety" or "residue" are herewith intended to define the residual portion of a given molecule once properly attached or conjugated, either directly or through a suitable linker and/or spacer, to the rest of the molecule.

The term "imaging agent" refers to a detectable entity that can be used to *in vitro* or *in vivo* visualize or detect

a biological element including cells, biological fluids and biological tissues originating from a live mammal patient, and preferably, human patient, as well as human body organ, regions or tissues, when the said detectable entity is used in association with a suitable diagnostic imaging technique.

*Targeting moiety (T)*

According to the invention, a targeting moiety (T) is a molecule that binds with particular selectivity to a biological target and facilitates the accumulation of the contrast agent in a specific tissue or part of the body. Generally, it is represented by a natural or synthetic molecule for use in biological systems. Such specific binding can be achieved through a ligand, such as for instance a small molecule, a protein, a peptide, a peptidomimetic, an enzyme substrate, an antibody or fragment thereof or an aptamer, interacting with a specific biological target expressed on the surface of the tissues or cells of interest.

Suitable biological targets for the compounds of the invention can be for instance an epithelial growth factor (EGF) receptor, such as EGFR or HER2; a vascular endothelial growth factor (VEGF) receptor, such as VEGFR1 or VEGFR2; a carbonic anhydrase (CA) enzyme, such as CAIX, CAII or CAXII; a mucin glycoprotein, such as MUC1; a glucose transporter, such as GLUT-1; a sodium-hydrogen antiporter, such as NHE1; a carcinoembryonic glycoprotein, such as the carcinoembryonic antigen (CEA); a chemokine receptor, such as the chemokine receptor type 4 (CXCR4); a cell adhesion molecule, such as ICAM, EPCAM, VCAM, E-Selectin, P-Selectin; the hepatocyte growth factor HGFR (c-met); a receptor for the transferrin; a ephrin receptor, such as EPHA2; a receptor for the folic acid, such as FR-alpha; a glycoprotein binding ialuronic acid, such as CD44; a bombesin receptor, such as BB1, BB2, BB3; a N-acetyl-L-aspartyl-L-glutamate (NAAG) peptidase, such as prostate-specific membrane antigen (PSMA); and, in particular, an integrin receptor, such as $\alpha_v\beta_3$, $\alpha_v\beta_5$, $\alpha_v\beta_6$ or $\alpha_5\beta_1$ integrin receptors.

For instance, integrin receptors targeting moieties are represented by linear or cyclic peptides comprising the sequence Arg-Gly-Asp (RGD). This tripeptide has high binding specificity for the receptor, being recognized as ligand by the family of the integrin receptors located in the cell membrane. In fact, it has been identified in some extracellular matrix glycoproteins, such as fibronectin or vitronectin, which exploit this RGD motif to mediate cell adhesion.

Therefore, linear and cyclic peptides and peptidomimetics containing the sequence Arg-Gly-Asp (RGD), such as for instance cRGD, cRGDfK, cRGDyK, cRGDfC, RGD-4C, RGD-2C, AH111585, NC100692, RGD-K5 (Kapp et al., Sci Rep, 2017, 7:3905), or their analogues and derivatives thereof, are a well known example of binding motif targeting cancer tissues on which cell membrane integrins are up-regulated compared to healthy tissues.

In one embodiment, the compounds of the invention can be conjugated to other small molecules, peptides, proteins or antibodies, such as for instance monoclonal antibodies already used for therapy. Small molecules containing the drug acetazolamide, such as for instance compounds 4a, 5a, 6a, 7a and 8a (Wichert et al., Nat Chem 2015, 7: 241-249), or their analogues and derivatives thereof, are examples of small moleculs targeting the enzyme CAIX. Linear and cyclic peptides and peptidomimetics, such as peptide GE11 (described in Li et al., FASEB J 2005, 19:1978-85) and/or peptide L1 (described in Williams et al., Chem Biol Drug Des 2018, 91:605-619), or their analogues and derivatives thereof, are examples of peptides targeting the epithelial growth factor receptor (EGFR). Among the proteins, derivatives of the epithelial growth factor (EGF) are examples of small protein targeting the epithelial growth factor receptor (EGFR). Among the antibodies, panitumumab and cetuximab are examples of monoclonal antibodies targeting the epithelial growth factor receptor (EGFR).

Preferably, the targeting ligands of the invention are able to selectively link tumor cells or tissues. In particular they are able to link tumors selected from brain cancer, breast cancer, head and neck cancer, ovarian cancer, prostate cancer, esophageal cancer, skin cancer, gastric cancer, pancreatic cancer, bladder cancer, oral cancer, lung cancer, renal cancer, uterine cancer, thyroid cancer, liver cancer, and colorectal cancer. In addition, the targeting ligands are able to link metastatic spreads of the above-mentioned cancers in tissues and organs different from the primary source. Furthermore, the targeting ligands are able to link pre-neoplastic lesions and dysplasia in different tissues and organs.

In one embodiment the targeting moiety can also be a chelator that complexes and tightly binds metals. Examples of such metal chelators are known to person skilled in the art and are reported in the prior art and can be represented for instance by molecules of DOTA, NODAGA, TETA, CB-TE2A, EDTA, DTPA, Deferoxamine, NOTA, AAZTA, etc. These chelators can be used to complex metals such as $Gd^{3+}$ or $Mn^{2+}$ and applied as contrast agents in magnetic resonance imaging. In a preferred embodiment, chelators can be used to complex radiometals such as Technitium, Indium, Aluminum Fluoride, Zirconium, Yttrium, Lutitium, Scandium, Attinium, Gallium or Copper, and find application for instance as imaging agents in analyses with positron emission tomography, single photon emission computed tomography and gamma-counting.

*Spacer* S

According to the invention, **S** is a spacer, optionally present, that separates the targeting moiety from the dye.

The presence of a spacer is particularly relevant for some embodiments where the targeting moiety and the dye risk to adversely interact with each other. Moreover, the presence of the spacer may be necessary when the dye is relatively large and may interfere with the binding of the targeting moiety to the target site.

The spacer can be either flexible (e.g., simple alkyl chains) or rigid (e.g., cycloalkyl or aryl chains) so that the dye is oriented away from the target. The spacer can also modify pharmacokinetic and metabolism of the conjugates of formula (I) used as imaging agents in a living organism.

Hydrophilic spacers may reduce the interaction with plasma proteins, reduce blood circulation time and facilitate excretion. For example, if the spacer is a polyethyleneglycol (PEG) moiety, the pharmacokinetics and blood clearance rates of the imaging agent *in vivo* may be altered. In such embodiments, the spacer can improve the clearance of the imaging agent from background tissue (i.e., muscle, blood) thus giving a better diagnostic image due to high target-to-background contrast. Moreover, the introduction of a particular hydrophilic spacer may shift the elimination of the contrast agent from hepatic to renal, thus reducing overall body retention.

Therefore, in one preferred embodiment, the spacer is selected from the group consisting of $-NH(CH_2)_pCOO-$, $-NH(CH_2CH_2O)_PCH_2CH_2COO-$ and $-NH(CH_2CH_2O)_pCH_2CH_2NH-$, wherein p is an integer between 0 and 20. Preferably p is 2, 6 or 12.

When not necessary, the spacer is preferably absent, i.e. m is 0 and S represents a direct bond.

The spacer, or alternatively the targeting moiety when the spacer is absent, can be connected in a compound of formula (I) at the R5 residue, represented by the linking moiety R5' in the compounds of formula (II). The linking groups of R5' are reactive functional groups such as carboxylic or carboxamido residues suitable for conjugating the dye to the targeting moiety by formation of a chemical bond.

For instance, when an amine-containing targeting moiety (T) is conjugated with a compound of formula (II) wherein R5' is a carboxylic acid, this carboxylic acid may be optionally activated before carrying out the conjugation through conversion in a more reactive form using an activating reagent, forming for example a N-hydroxy succinimide (NHS) ester or a mixed anhydride. Then, to obtain the corresponding compound of formula (I), the amine-containing targeting moiety is treated with the resulting activated acid to form an amide linkage. Typically, this reaction is carried out in aqueous buffer.

Otherwise a direct conjugation using the "non-activated" carboxylic acid may be performed.

Similarly, when the linking group of R5' is a carboxamido group, the procedure for attachment of the suitable targeting moiety is analogous, but no activation step of the linker is generally required and the dye and targeting moiety are treated directly.

The compounds of the above formula (I) or (II) may have one or more asymmetric carbon atoms, otherwise referred to as chiral carbon atoms, and may thus give rise to diastereomers and optical isomers. Unless otherwise provided, the present invention further includes all such possible diastereomers as well as their racemic mixtures, their substantially pure resolved enantiomers, all possible geometric isomers, and pharmaceutically acceptable salts thereof.

The present invention further relates to compounds of the above formula (I) or (II) in which the functional groups of R3 and/or R5', e.g. the sulfonyl, carboxyamino or carboxylic groups, may be in the form of a pharmaceutically acceptable salt.

In one embodiment, the invention relates to a compound of formula (I) or (II) wherein R1 and R2 are independently a group -CO-NH-Y wherein Y, optionally the same moiety for R1 and R2, is selected from a linear or branched alkyl, cycloalkyl and heterocyclyl, substituted with from two to five hydroxyl groups.

In a preferred embodiment the invention relates to a compound of formula (I) or (II) wherein n is 3, R3 is alkyl substituted by $-SO_3H$, and R1 and R2 can be the same or different and are selected from the group consisting of

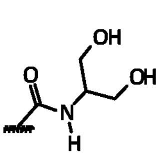 (iii), 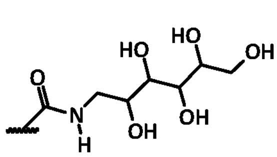 (iv), 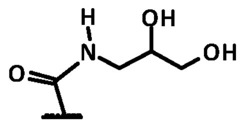 (v), 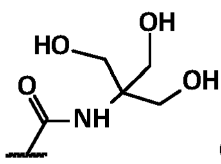 (vi),

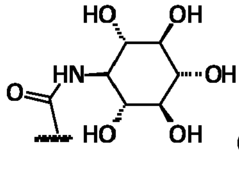 (vii) and 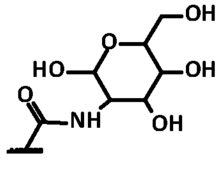 (viii).

More preferably n is 3, R3 is alkyl substituted by $-SO_3H$, and R1 and R2 are the same.

In another embodiment of the invention m is 0 and the spacer (S) is represented by a direct bond or m is 1 and the spacer is an hydrophilic moiety comprising alkyl, cycloalkyl or aryl groups.

Preferably, the spacer is selected from $-NH(CH_2)_pCOO-$, $-NH(CH_2CH_2O)_pCH_2CH_2COO-$ and $-NH(CH_2CH_2O)_pCH_2CH_2NH-$, wherein p is an integer between 0 and 20. Preferably p is 2, 6 or 12.

In a further embodiment T is a targeting moiety selected from a small molecule, a protein, a peptide, a peptidomimetic, an enzyme substrate, an antibody or any fragment thereof and an aptamer.

Preferably T is represented by a peptide, and in particular by a moiety interacting with an integrin receptor, such as $\alpha_v\beta_3$, $\alpha_v\beta_5$, $\alpha_v\beta_6$, $\alpha_5\beta_1$ and the like, preferably with $\alpha_v\beta_3$ integrin receptor.

In another preferred embodiment, the invention relates to a compound of formula (I) or (II) wherein n is 3, R3 is $C_4$-alkyl substituted with $-SO_3H$ and both R1 and R2 are a group (iv) as defined above, otherwise represented by the following formula (Ia) or (IIa) respectively:

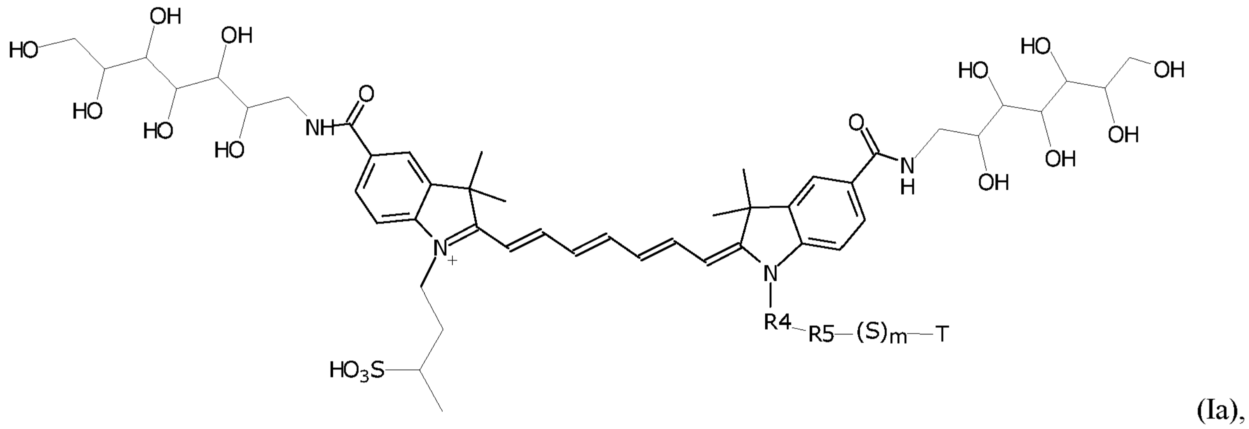

(Ia),

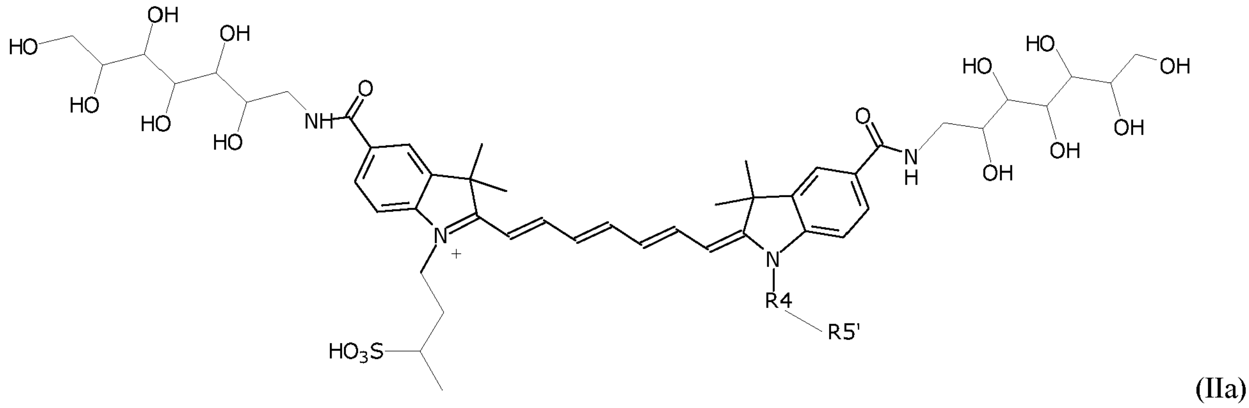

(IIa)

wherein R4, R5, R5', S, m and T are as defined above.

In another embodiment, the invention relates to a compound of formula (I) or (II) wherein n is 1 or 2. Especially preferred are the compounds of formula (I) or (II) listed in Table Ia and Ib.

Table Ia – *Preferred compounds of formula (I)*

| |
|---|
| 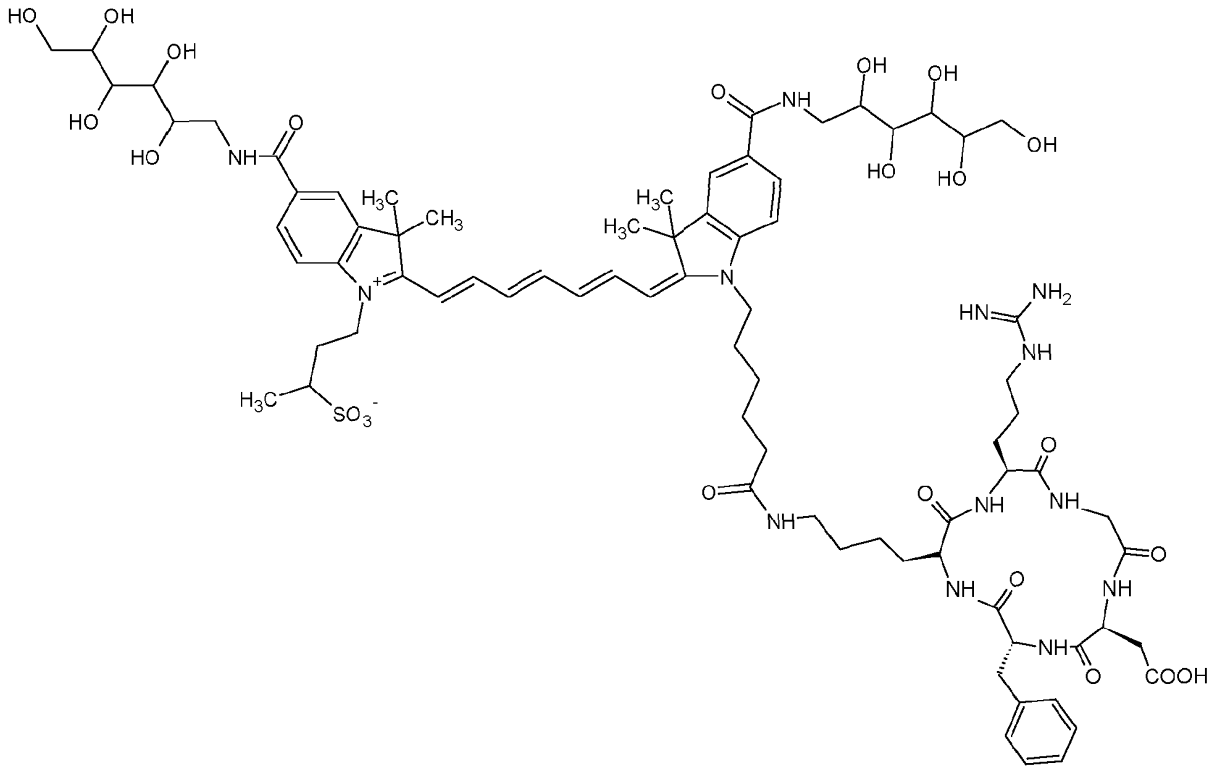<br>Compound 1 |
| 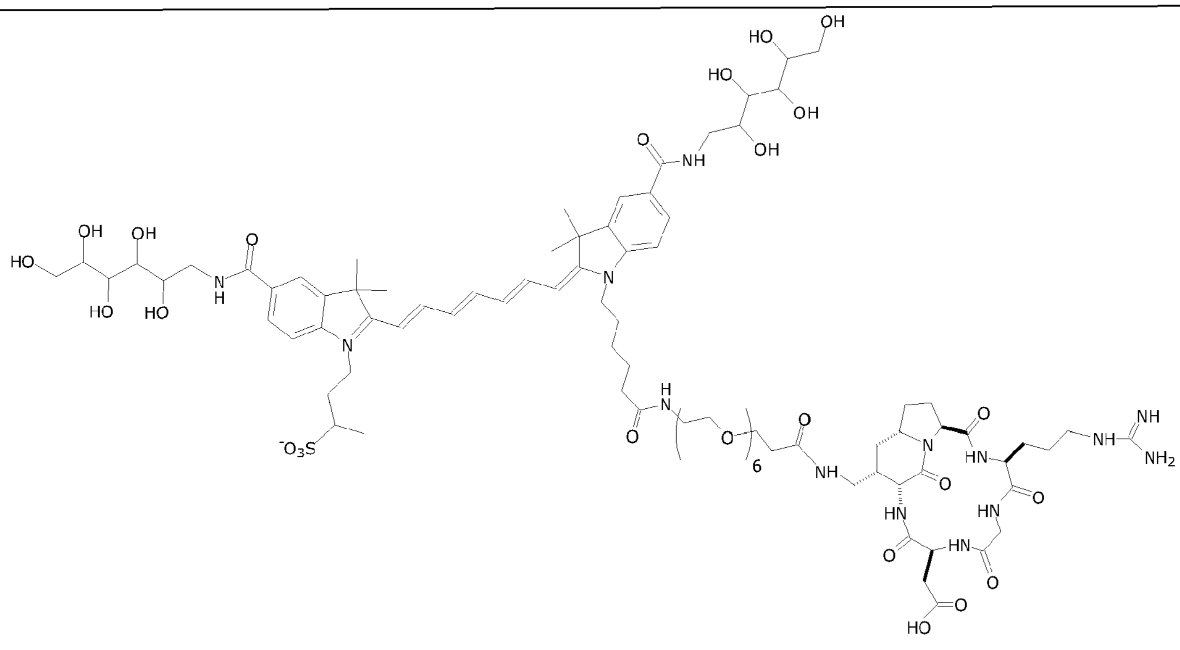<br>Compound 2 |

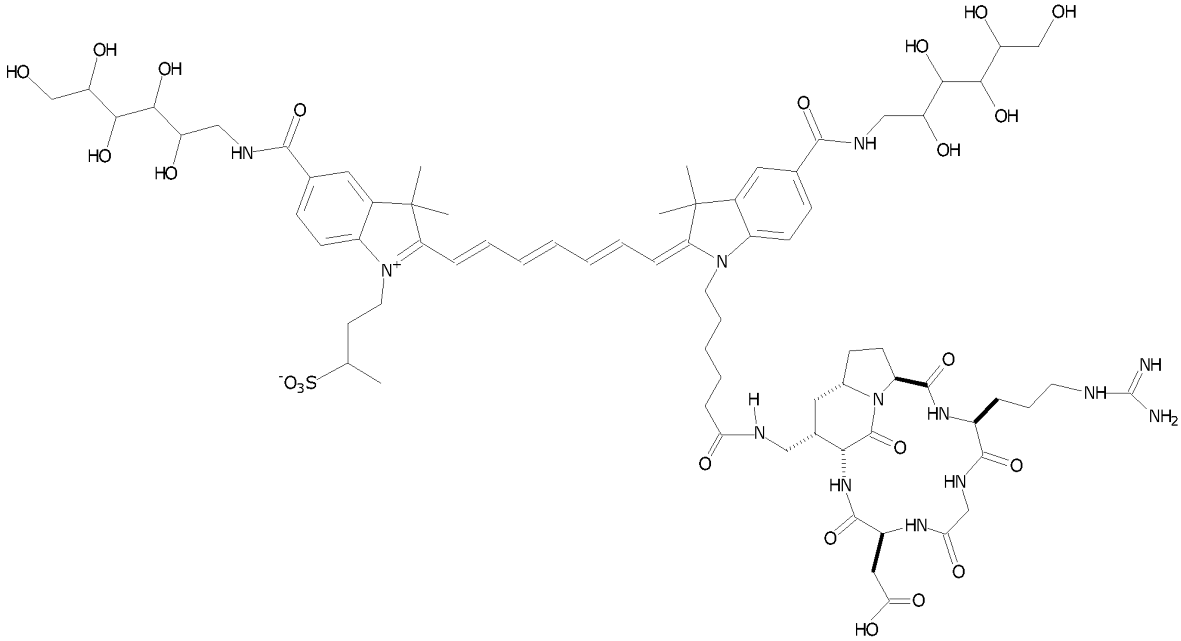

Compound 3

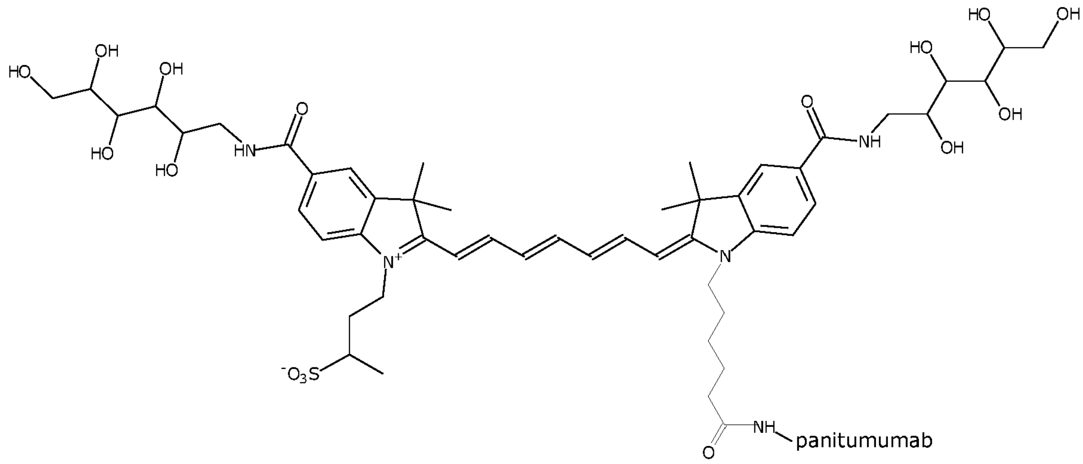

Compound 9

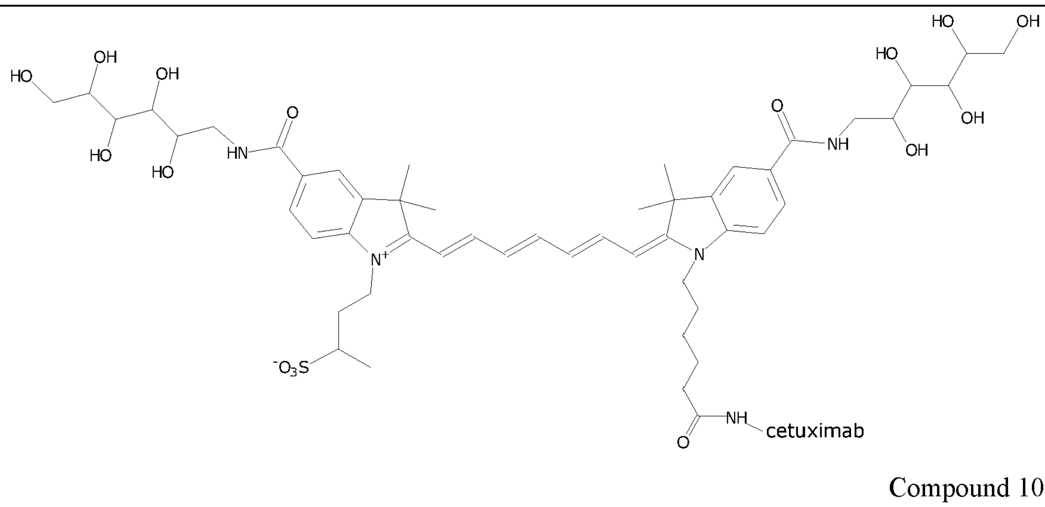

Compound 10

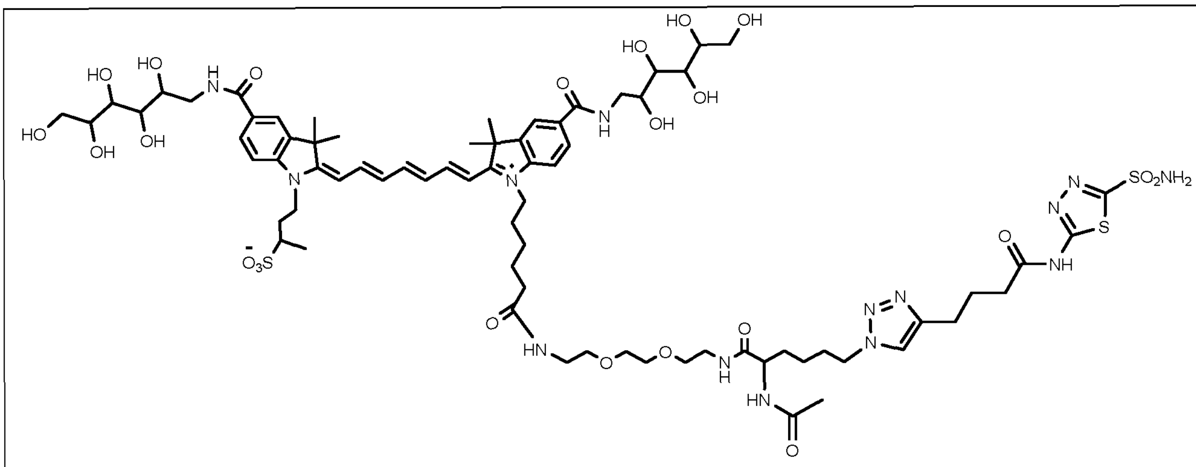

Compound 11

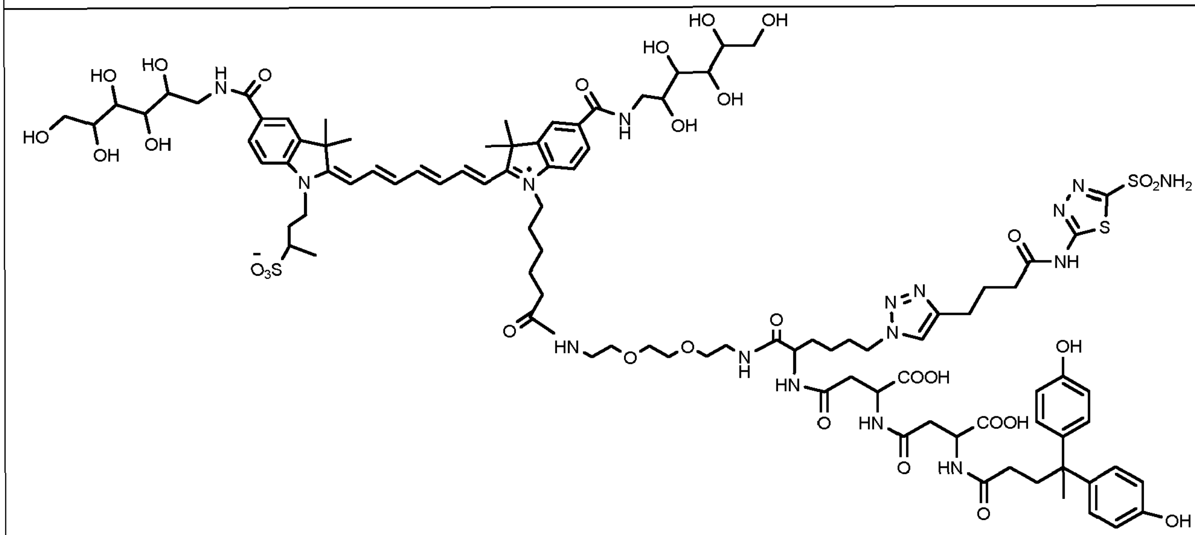

Compound 12

Table 1b – *Preferred compounds of formula (II)*

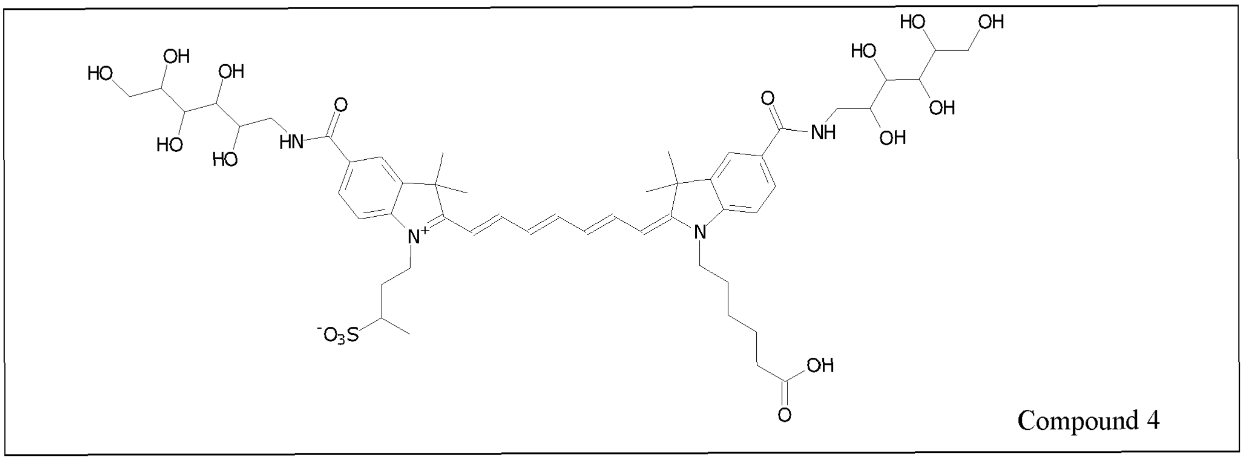

Compound 4

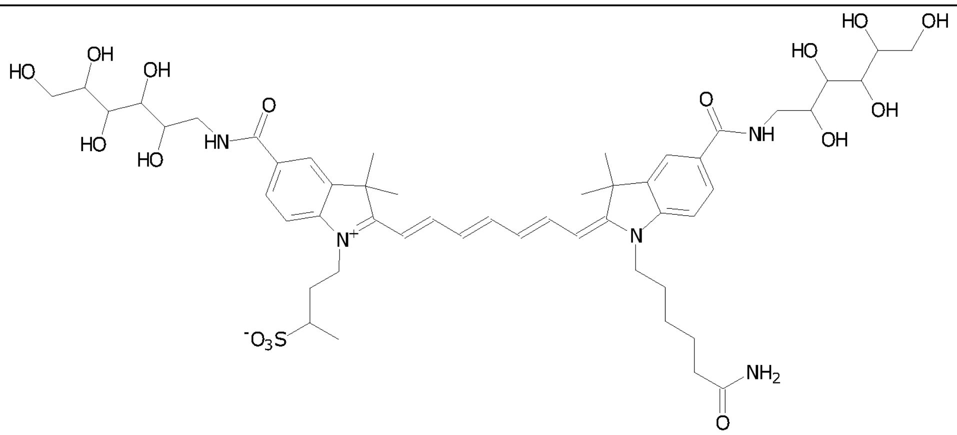

Compound 5

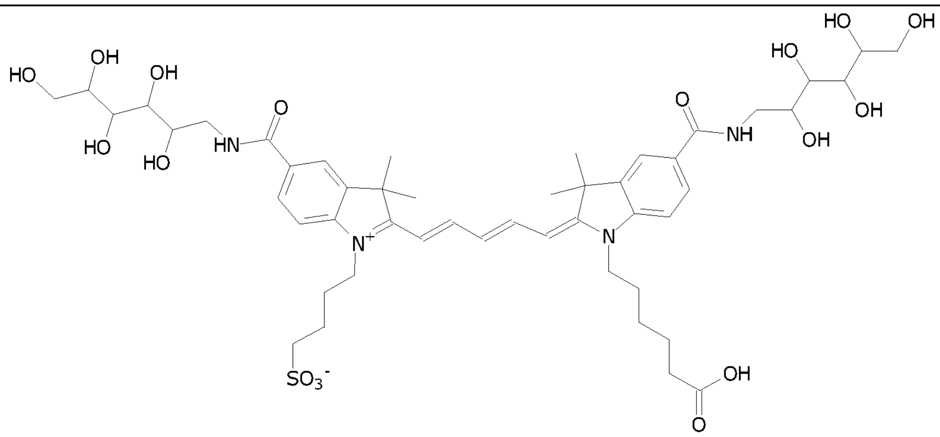

Compound 6

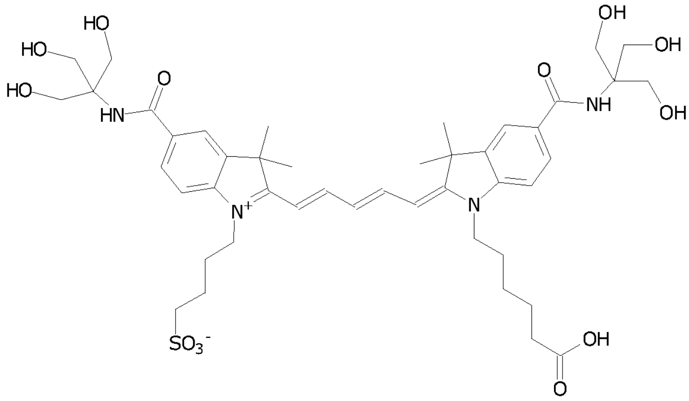

Compound 7

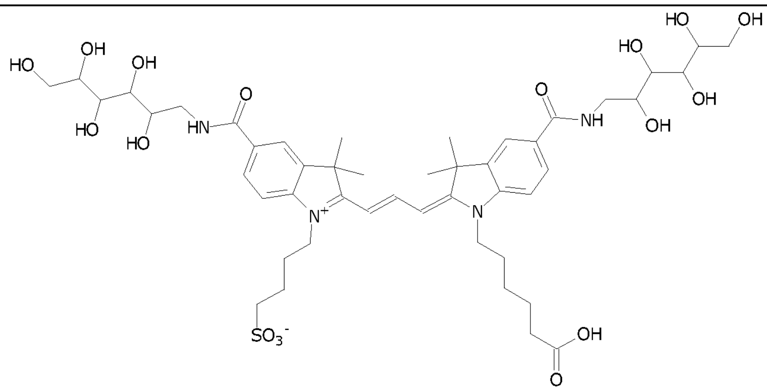

Compound 8

The present invention is also directed to methods for synthesizing the compounds of formula (I) and (II) prepared as illustrated in the following of the description. The compounds of the invention are useful as imaging agents in the detection of tumors in both humans and animals. Accordingly, the invention provides the compounds of formula (I) as defined above for use as fluorescent probes for the detection and demarcation of a tumor margin in guided surgery of an individual patient, in particular wherein said tumor is a tumor showing a reduced or variable over-expression of integrin receptors. Preferably the imaged subject is a human.

The invention also provides a compound of formula (I) for use as fluorescent probe as defined above, wherein the detection and demarcation of the tumor margin is carried out under NIR radiation.

A further aspect of this invention relates to a pharmaceutical composition comprising a conjugate of formula (I) or a dye of formula (II) as defined above, or a salt thereof, and one or more pharmaceutically acceptable adjuvants, excipients or diluents.

Another aspect of this invention relates to a diagnostic kit comprising a conjugate of formula (I) as defined above. In addition, the kit can contain additional adjuvants for implementing the optical imaging. These adjuvants are, for example, suitable buffers, vessels, detection reagents or directions for use. The kit preferably contains all materials for an intravenous administration of the compounds of the invention.

The compounds of the invention may be administered either systemically or locally to the organ or tissue to be imaged, prior to the imaging procedure. For instance, the compounds can be administered intravenously. In another embodiment they may be administered parenterally or enterally. The compositions are administered in doses effective to achieve the desired optical image of a tumor, tissue or organ, which can vary widely, depending on the compound used, the tissue subjected to the imaging procedure, the imaging equipment being used and the like. The exact concentration of the imaging agents is dependent upon the experimental conditions and the desired results, but typically may range between 0.000001 mMto 0.1 mM. The optimal concentration is determined by systematic variation until satisfactory results with minimal background fluorescence are obtained. Once administered, the imaging agents of the invention are exposed to a light, or other form of energy, which can pass through a tissue layer. Preferably the radiation wavelength or waveband matches the excitation wavelength or waveband of the photosensitizing agent and has low absorption by the non-target cells and the rest of the subject, including blood proteins. Tipically, the optical signal is detectable either by observation or instrumentally and its response is related to the fluorescence or light intensity, distribution and lifetime.

## *DESCRIPTION OF THE SYNTHESES*

The preparation of the compounds of formula (I) or (II), as such or in the form of physiologically acceptable salts, represents a further object of the invention. The cyanine dyes and dye-conjugates of the invention can be prepared for instance according to the methods described in the following sections and in the experimental part. A general teaching about the preparation of cyanine dyes can be found in Mujumdar R.B. et al., Bioconjugate Chem. 1993, 4(2), 105-111, which relates to the synthesis and labeling of sulfoindocyanine dyes. However, the cyanines of the present invention are characterized by a specific functionalization pattern not present in the compounds of the art, for which the set up of a proper synthetic approach was required. In fact, unlikely other known cyanines, the compounds of the invention bears even three functional moieties (carboxylic acid or amino/amide groups) to be derivatized in different ways, so that the use of protecting groups was necessary in most cases to direct the reactions on the desired functional group.

It is known that difficulties can arise when manipulating the cyanines at the strong pH and temperature conditions necessary for the removal of the protecting groups, since tha stability of the polymethine scaffold can be compromised in some cases, with severe degradation of the dyes.

Moreover, further obstacles can be encountered due to a possible hydrolysis and degradation of the amide groups R1 and R2 when deprotecting the carboxylic group of R5 (typically, amide derivatives can hydrolyze in concentrated alkaline medium, see for instance Yamana et al, Chem. Pharm. Bull., 1972, 20(5), 881-891). Contrary to what was expected, the compounds of the invention have been found very stable at basic pH (i.e. at about pH 11) and none or negligible degradation has been observed during the removal of the protecting groups.

In one preferred embodiment, the protective group for the moiety R5' is an ester group.

More preferably, an ethyl ester group can be advantageously used.

### Preparation of cyanine dyes of formula (II)

According to the invention, compounds of formula (II) can be prepared through a general synthetic process as reported in the following Scheme 1, for the embodiments wherein R1 is the same of R2, or in Scheme 2, for the embodiments wherein R1 and R2 have a different meaning.

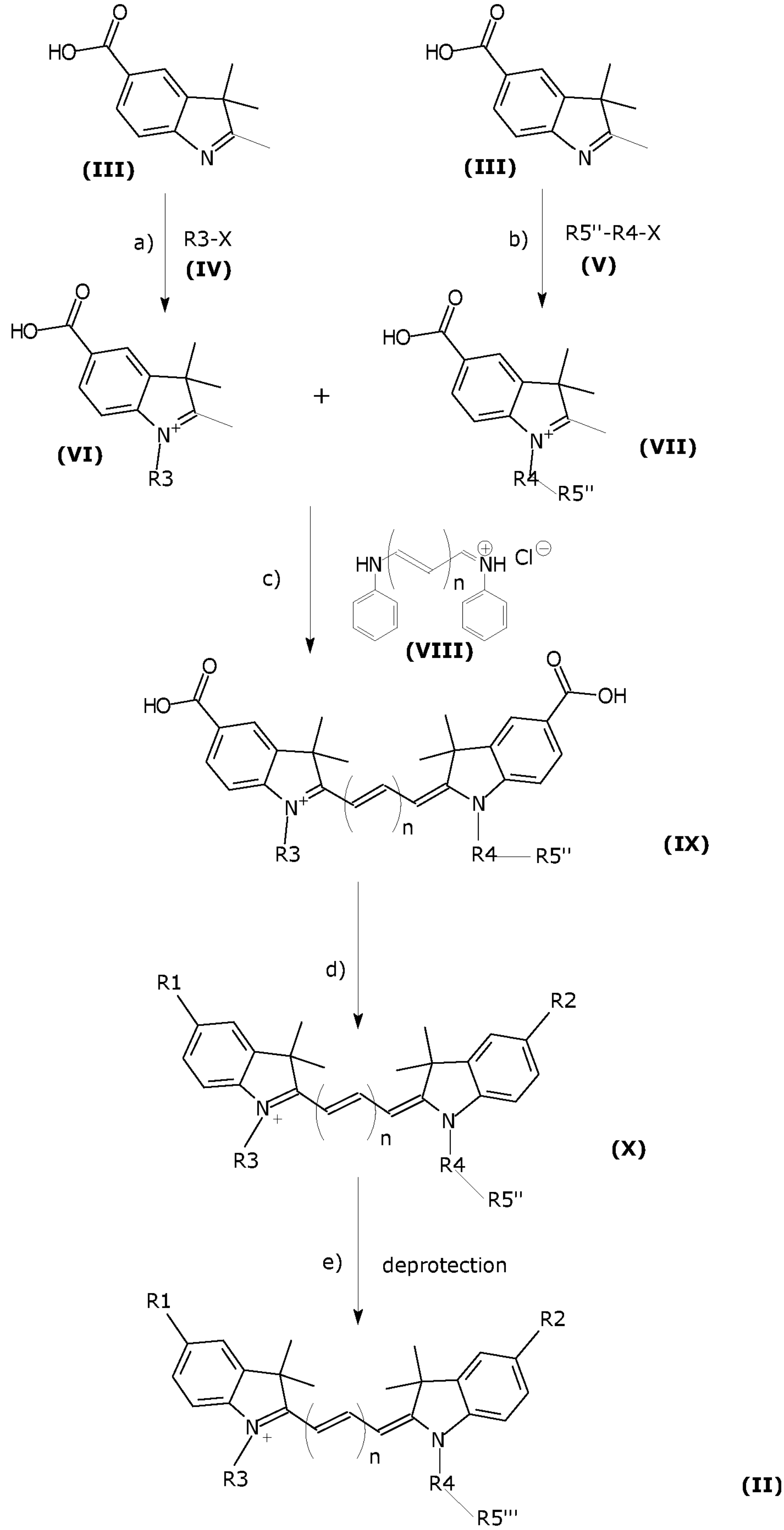


Scheme 1

In the above Scheme 1, R1, R2, R3, R4, and n are as defined above, R5" is -COOPg or -CONHPg, wherein Pg is a protecting group, R5‴ is -COOH or $-CONH_2$ and X is a suitable leaving group such as halogen, mesylate or triflate.

Accordingly, a process of the present invention comprises the following steps:

a) treating an amount of 5-carboxy-2,3,3-trimethylindolenine **(III)** with a nucleophile R3-X **(IV),** wherein R3 is as defined above and X is a suitable leaving group, such as an halide group, or R3-X represent a $C_1$-$C_6$-alkanesultone molecule, thus obtaining the intermediate **(VI);**

b) treating another amount of 5-carboxy-2,3,3-trimethylindolenine **(III)** with a nucleophile R5"-R4-X **(V),** wherein R5" and R4 are as defined above and X is a suitable leaving group such as an halide group, thus obtaining the intermediate **(VII);**

c) reacting the intermediate **(VI)** and the intermediate **(VII)** together with the reagent **(VIII),** to obtain the cyanine scaffold **(IX),** wherein n, R3, R4 and R5" are as defined above;

d) derivatizing the carboxylic acid groups on the indolenic rings with a polyhydroxylated amine, such as for instance glucamine, meglumine, glucosamine, trometamol, serinol or isoserinol;

e) optionally, removing any protecting group from the resulting intermediate **(X)** and isolating the compound of formula (II) or the salt thereof.

According to steps a) and b) the reaction of derivative (III) with a nucleophile R3-X (IV) or R5"-R4-X (V) can be carried out neat or in high boiling solvents, such as butyrronitrile, sulfolane, 1,2-dichlorobenzene, dimethylacetamide, dimethylformamide or dimethylsulfoxide, stirring the solution at high temperature, for instance between 90°C and 180°C, for several hours, tipically from 12 hours to 5 days.

According to step c) the reaction can be performed using the Vilsmeier reagent in the bis anilido form (as reported in Scheme 1) or in the bis aldehyde form. The reaction can be carried out in several solvents such as for example ethanol, methanol, acetic anhydride or acetic acid, with or without the addition of different bases, such as trimethylamine, pyridine, sodium acetate, potassium acetate etc., stirring the mixture at different temperatures ranging from 45°C to 120°C for several hours (typically 2-24 hours).

According to step d) the reaction can be performed using several coupling agents, such as TBTU, HBTU, HATU, PyBOP, DCC, DSC, DCC-NHS and several organic bases such as TEA, DIPEA, NMM, pyridine, etc., in solvents such as dimethylformamide, dimethylacetamide, dimethylsulfoxide, acetonitrile etc, at room temperature for a suitable time ranging from 30 minutes to several hours.

According to optional step e), any protecting group of intermediate (X) is removed from the moiety R5" according to the known procedures, described for instance in T.W. Green and P.G.M.Wuts, Protective Groups in Organic Synthesis, Wiley, N.Y. 2007, 4th Ed., Ch. 5.

Alternatively, as said above, when R1 is different from R2 a compound of formula (II) can be prepared as illustrated in the following Scheme 2:

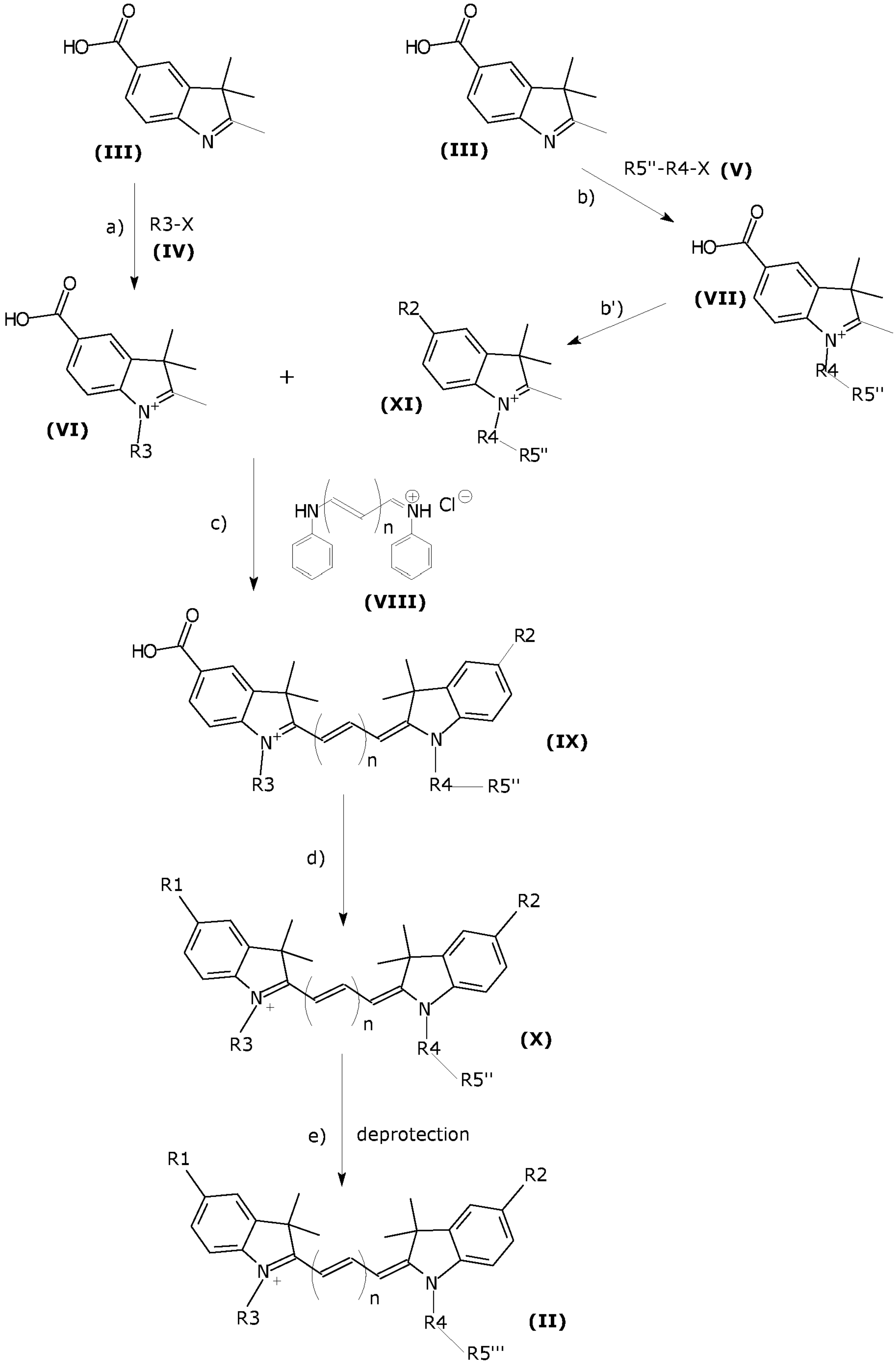
(III)
(III)
R5''-R4-X (V)
b)
a) R3-X (IV)
(VII)
b')
(VI)
+
(XI)
c)
(VIII)
(IX)
d)
(X)
e) deprotection
(II)

Scheme 2

wherein R1, R2, R3, R4, and n are as defined above, R5" is -COOPg or -CONHPg, wherein Pg is a protecting group, R5‴ is -COOH or $-CONH_2$ and X is a suitable leaving group such as halogen, mesylate or triflate. According to Scheme 2, a polyhydroxylated amine is reacted in step b') with an intermediate (VII) to form the intermediate (XI), bearing the residue -R2, before carrying out the Vilsmeier reaction of step c) to form the cyanine (IX). The other different residue -R1 can be added afterwards on the second indolenine ring. Alternatively, the group -R1 may also be introduced in intermediate (VI), in a corresponding step a'), before performing step c).

Accordingly, step b'), and/or optionally a'), is carried out as described above for step d).

In a further embodiment, a compound of formula (II), prepared according to the processes of the invention, can be conveniently converted into another compound of formula (II) by operating according to well-known synthetic conditions, the following being examples of possible conversions:

f) converting a compound of formula (II) wherein R5‴ is -COOH, i.e. a compound of formula (IIb), into a corresponding compound of formula (II) wherein R5‴ is $-CONH_2$, i.e. a compound of formula (IIc):

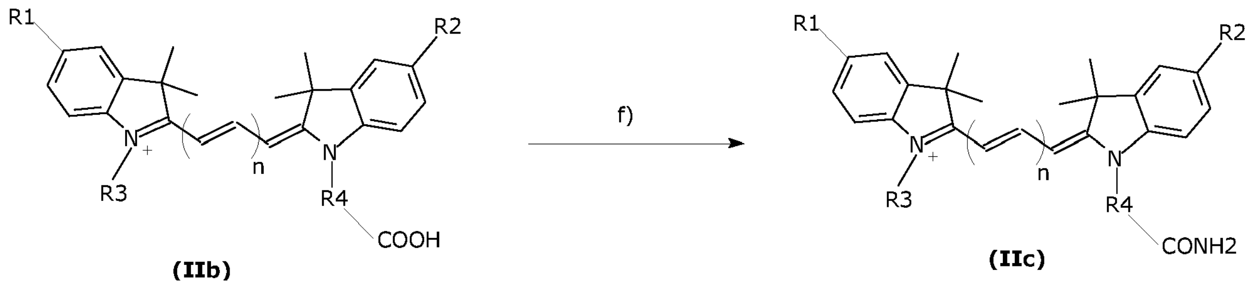


**(IIb)** **(IIc)**

According to step f), the conversion of a carboxylic acid of formula (IIb) into the corresponding carboxamide of formula (IIc) can be accomplished in a variety of ways and experimental conditions, which are widely known in the art for preparation of carboxamides. As an example, the carboxylic acid can be first converted in a suitable activated ester and then reacted with an ammonium salt, such as $NH_4Cl$, preferably in the presence of a coupling agent, such as HBTU.

**Preparation of conjugate compounds of formula (I)**

The cyanine derivatives of formula (II), or salts thereof, can be then conjugated with a suitable targeting moiety, optionally with the insertion of a spacer, to obtain the corresponding compounds of formula (I), as reported in Scheme 3:

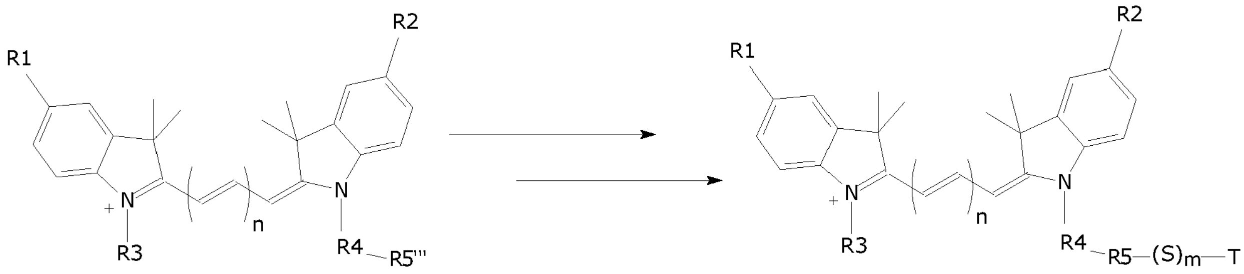

**(II)** **(I)**

Scheme 3

The conjugation can be accomplished following different procedures known in the art, such as for instance via direct coupling, wherein the functional group R5‴ is directly reacted with a nucleophilic residue of the targeting moiety, or optionally with the spacer, or by previous activation, wherein the functional group of R5‴, typically an acid, is transformed in a more reactive group, e.g. an ester such as NHS, before the coupling.

If a compound of the formula (I) or (II) prepared according to the processes described above is obtained as mixture of isomers, their separation using conventional techniques into the single corresponding isomer of the formula (I) or (II) is within the scope of the present invention.

The final compounds may be isolated and purified using conventional procedures, for example chromatography and/or crystallization and salt formation.

A compound of formula (I) or (II) as defined above can be coverted into a pharmaceutically acceptable salt. The compounds of formula (I) or (II) as defined above, or the pharmaceutically acceptable salt thereof, can be subsequently formulated with a pharmaceutically acceptable carrier or diluent to provide a pharmaceutical composition.

## DESCRIPTION OF THE DRAWINGS

Figure 1 represents the fluorescence signal decay in the healthy muscle tissue after intravenous administration of three doses (0.3 nmol/mouse, circles; 1 nmol/mouse, squares; 3 nmol/mouse, triangles) of compound 1 in Balb/c nu/nu mice (n=5/group).

Figure 2 represents the fluorescence decay (left y-axis) in the tumor (black circles) and muscle tissue (white circles) after iv administration of 1 nmol of compound 1 in Balb/c nu/nu mice implanted with 10 million of U87MG cells, and tumor-to-background ratio (squares, right y-axis) over time.

Figure 3 shows the tumor-to-background ratio (TBR) of compound 1 administered at the dose of 3 nmol/mouse in Balb/c nu/nu mice implanted with 2.5 million of Detroit-562 cells (n=5).

Figure 4 represents the fluorescence decay (left y-axis) in the tumor (black circles) and muscle tissue (white circles) after administration of 1 nmol of compound 1 in Athymic nude mice implanted with 5 million of HT-29 cells (n=5), and tumor-to-background ratio (squares, right y-axis) over time.

All data are expressed as mean ± standard deviation.

## EXPERIMENTAL PART

The invention and its particular embodiments described in the following part are only exemplary and not to be regarded as a limitation of the present invention: they show how the present invention can be carried out and are meant to be illustrative without limiting the scope of the invention.

*Materials and Equipment*

All chemicals and solvents used for the reactions were reagent grade. Analytical grade solvents were used for chromatographic purifications. Most of the reagents, unless reported otherwise, are commercial products, including the targeting moieties (e.g. Panitumumab (Vectibix, Amgen; CAS Registry Number of the active substance: 339177-26-3); Cetuximab (Erbitux, Merck; CAS Registry Number of the active substance: 205923-56-4)).

All synthesized compounds were purified by reverse phase chromatography (RP-HPLC) and characterized by mass spectroscopy using a LC/MS instrument equipped with a UV-VIS detector and an ESI source.

Analysis were performed with a Waters Atlantis dC18 5μm, 4.6 × 150 mm column using a gradient of phase A $CH_3COONH_4$ 10mM and phase B acetonitrile. Measured mass/charge ratios are listed for each compound. A dual-beam UV-VIS spectrophotometer (Lambda 40, Perkin Elmer) was used to determine the absorbance (Abs) of the compounds of the invention. Emission/excitation (Em/Ex) spectra and absolute fluorescence quantum yield (ϕ) measurements were carried out on a spectrofluorometer (FluoroLog-3 1IHR-320, Horiba Jobin Yvon) equipped with an F-3018 integrating sphere accessory. The measurements were performed using an excitation wavelength at maximum absorbance of different dyes, and the sample was excited with a 450W Xenon Light Source. Detection was performed by photomultiplier tubes (PMT-NIR) cooled detector or by TBX-04 detector. Dye solutions were carefully prepared to have an absorbance lower than 0.1 (optical densities) to minimize re-absorption phenomena.

In vivo imaging experiments were performed using the IVIS Spectrum In Vivo Imaging System (Perkin Elmer Inc.). The system is equipped with with 10 narrow band excitation filters (30nm bandwidth) and 18 narrow band emission filters (20 nm bandwidth) spanning 430 - 850 nm.

*List of abbreviations*

| | |
|---|---|
| DCC | N,N'-dicyclohexylcarbodiimide |
| DIPEA | N,N-Diisopropylethylamine |
| DMF | Dimethylformamide |
| DMSO | Dimethyl sulfoxide |
| DSC | N,N'-Disuccinimidyl carbonate 1-[Bis(dimethylamino)methylene]-1H-1,2,3 -triazolo [4,5 -b]pyridinium 3 -oxid |
| HATU | hexafluorophosphate |
| HBTU | O-Benzotriazol-1-yl-N,N,N',N'-tetramethyluronium hexafluorophosphate |
| HPLC | High performance liquid chromatography |

| | |
|---|---|
| PBS | Phosphate buffered saline |
| NHS | N-hydroxysuccinimide |
| NMM | N-methylmorpholine |
| RT | Room temperature |
| PyBOP | (benzotriazol-1-yloxy)tripyrrolidinophosphonium hexafluorophosphate |
| TEA | Triethylamine |
| TBTU | 2-(1H-Benzotriazole-1-yl)-1,1,3,3-tetramethyluronium tetrafluoroborate |
| TSTU | O-(N-Succinimidyl)-1,1,3,3-tetramethyluronium tetrafluoroborate |
| μL | Microliter |
| μM | Micromolar |
| $t_R$ | Retention time (HPLC) |
| cRGDfK | Cyclo-(Arg-Gly-Asp-D-Phe-Lys) |
| aza-cRGD-$NH_2$ | Aza-cyclo-(Arg-Gly-Asp) |

The abbreviations for individual amino acids residues are conventional: for example, Asp or D is aspartic acid, Gly or G is glycine, Arg or R is arginine. The amino acids herein referred to should be understood to be of the L-isomer configuration unless otherwise noted.

**Example 1: Synthesis of Compound 4**

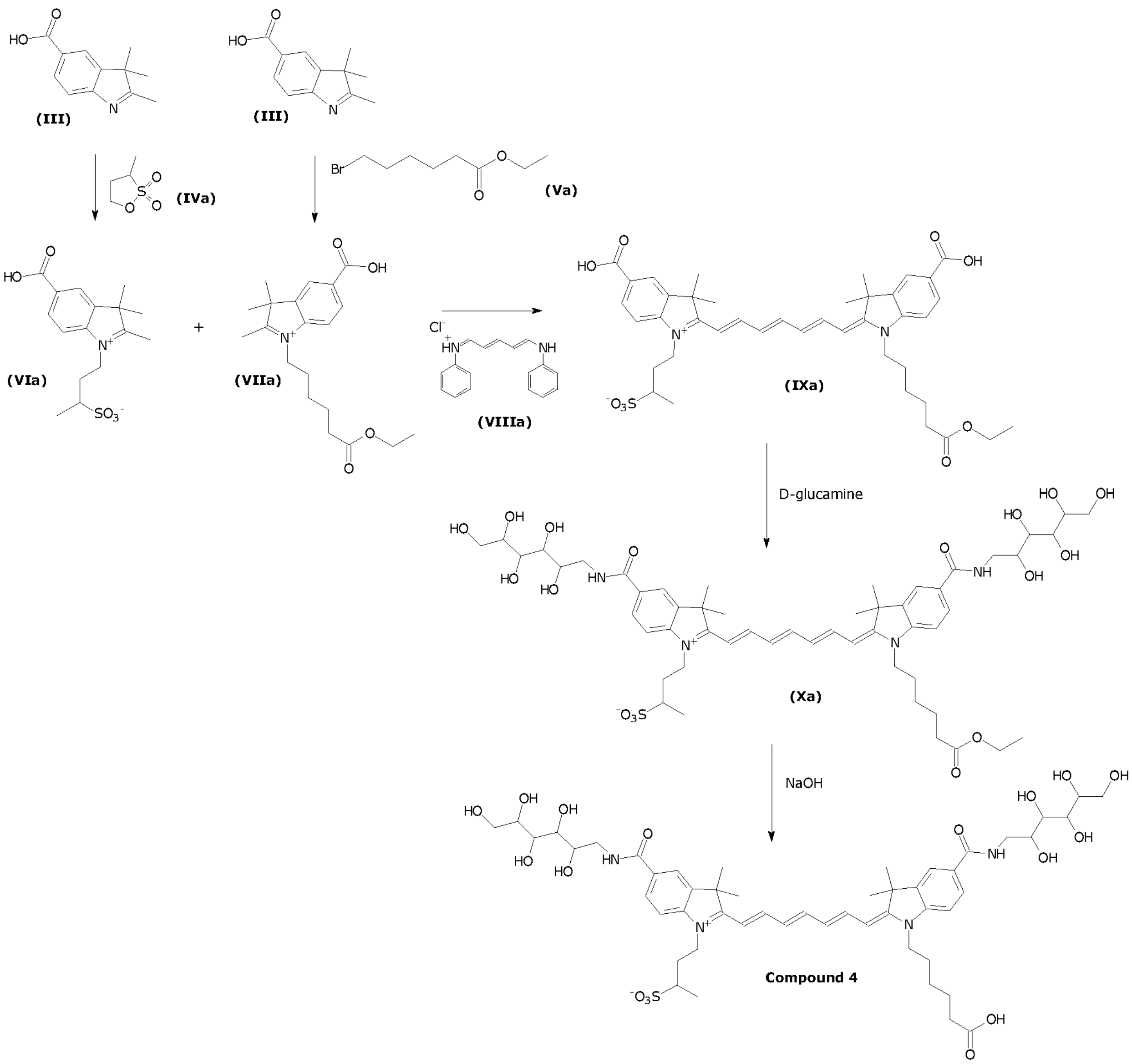


(III) (III) (IVa) (Va) (VIa) (VIIa) (VIIIa) (IXa) (Xa) Compound 4

*Preparation of intermediate (VIa), Step a*

In a round bottom flask, 5-carboxy-2,3,3-trimethylindolenine (1.50 g, 7.38 mmol), 2,4-butanesultone (1.11 g, 8.15 mmol) and butyronitrile (2 g) were added. The mixture was heated at 120 °C for 2 days. The solid was then treated with dry acetone, filtered under vacuum and washed twice with acetone. The pink solid was dried under vacuum and used with no further purification (2.40 g, 96%). HPLC purity at 270 nm 94%, MS: [M + H]+ 339.2.

*Preparation of intermediate (VIIa), Step b*

In a round bottom flask intermediate III (1.5 g, 7.38 mmol), ethyl 6-bromohexanoate (1.97 g, 8.85 mmol) and sulfolane (6.8 g) were added. The suspension was heated at 90°C for 3 days. Then, ethyl acetate (25 mL) was added, the purple dispersion was stirred at RT for 15 minutes and filtered under vacuum. The solid was washed with ethyl acetate three times, then it was redispersed in fresh ethyl acetate (15 mL), stirred at RT for 15 minutes, filtered under vacuum and washed with ethyl acetate. This operation was repeated for other two times. Finally, the pink /violet solid, containing both the desired product and the starting material, was dried under vacuum. It was then dispersed in acetonitrile (4 mL), the dense dispersion was stirred at RT for 15 minutes and filtered. The solid was washed with a few volume of cold acetonitrile. The mother liquor contained prevalently the desired product, whereas the solid contained the starting material. The acetonitrile was evaporated under vacuum and the solid was dispersed again in acetonitrile (4 mL), stirred for 15 minutes, filtered and the solid was washed with cold acetonitrile. The solution was concentrated under vacuum

obtaining a pink/violet solid with a HPLC purity of 92% at 270 nm (1.25 g as bromine salt, 40%). MS: [M + H]+ 346.5.

*Preparation of intermediate (IXa), Step c*

In a round bottom flask glutaconaldehyde dianyl hydrochloride (0.48 g, 1.4 mmol), acetic anhydride (47 mL) and acetic acid (13 mL) were added. The orange-brown solution was heated at 60°C for 3 h. The reaction mixture was then cooled down to RT and the solution of intermediate (VIa) (0.60 g, 1.4 mmol) in ethanol (10 mL) was dropped at RT in about 20 minutes. The red solution was heated at 35°C for 2 h, then the temperature was increased at 50°C and a solution of intermediate (VIIa) (0.48 g, 1.4 mmol) and sodium acetate (0.11 g, 1.3 mmol) in ethanol (19 mL) was added. Immediately after, pyridine (2.0 mL) was added. The reaction mixture was heated at 60°C for 1.5 h, then solvents were evaporated under vacuum and the dark oil was precipitated in cold water (100 mL). The dark green solid was filtered and washed with cold water. Then, it was dissolved in the minimum amount of ethanol and this solution was dropped under stirring into ethyl acetate (400 mL). The solid was filtered, washed with ethyl acetate, dissolved in dichloromethane and purified on silica gel with a slow dichloromethane-methanol gradient. Fractions containing the pure product were combined, evaporated under vacuum and dried obtaining a green-blue solid (350 mg, 33% yield). HPLC purity at 756 nm 94%, MS: [M + H]+ 747.3.

*Synthesis of Compound 4, Steps J and e*

In a dried round bottom flask, dry DIPEA (115 μL, 0.56 mmol) was added to a solution of intermediate (IXa) (150 mg, 70% purity, 0.14 mmol) in DMF (2 mL). After 30 minutes of stirring under nitrogen flow, a solution of TBTU (309 mg, 0.67 mmol) in DMF (2 mL) was added. After 1 h, a suspension of D-glucamine (87 mg, 0.336 mmol) in DMF (2 mL) was added. The mixture was stirred for 2 h at RT, then concentrated and purified on a pre-packed C18 silica column with a water-acetonitrile gradient. Fractions containing the pure product were collected, concentrated and freeze-dried, obtaining a green-blue powder (95.8 mg, 64% yield). HPLC purity at 756 nm 97%, MS: [M + Na]+ 1095.3.

The intermediate (Xa) thus obtained (95 mg, 0.089 mmol) was solubilized in water (10 mL, pH 6.35), then 2N NaOH was added until pH 11.14. The solution was stirred at room temperature maintaining pH 11 through addition of 0.1N NaOH. After 40 h the reaction was completed, the mixture was neutralized with 1N HCl and purified on a pre-packed C18 silica column with a water-acetonitrile gradient. Fractions containing the pure product were collected, concentrated and freeze-dried, obtaining a green-blue powder (81 mg, 86% yield). HPLC purity at 756 nm 98%, MS: [M + H]+ 1045.3.

**Example 2: Synthesis of compound 5**

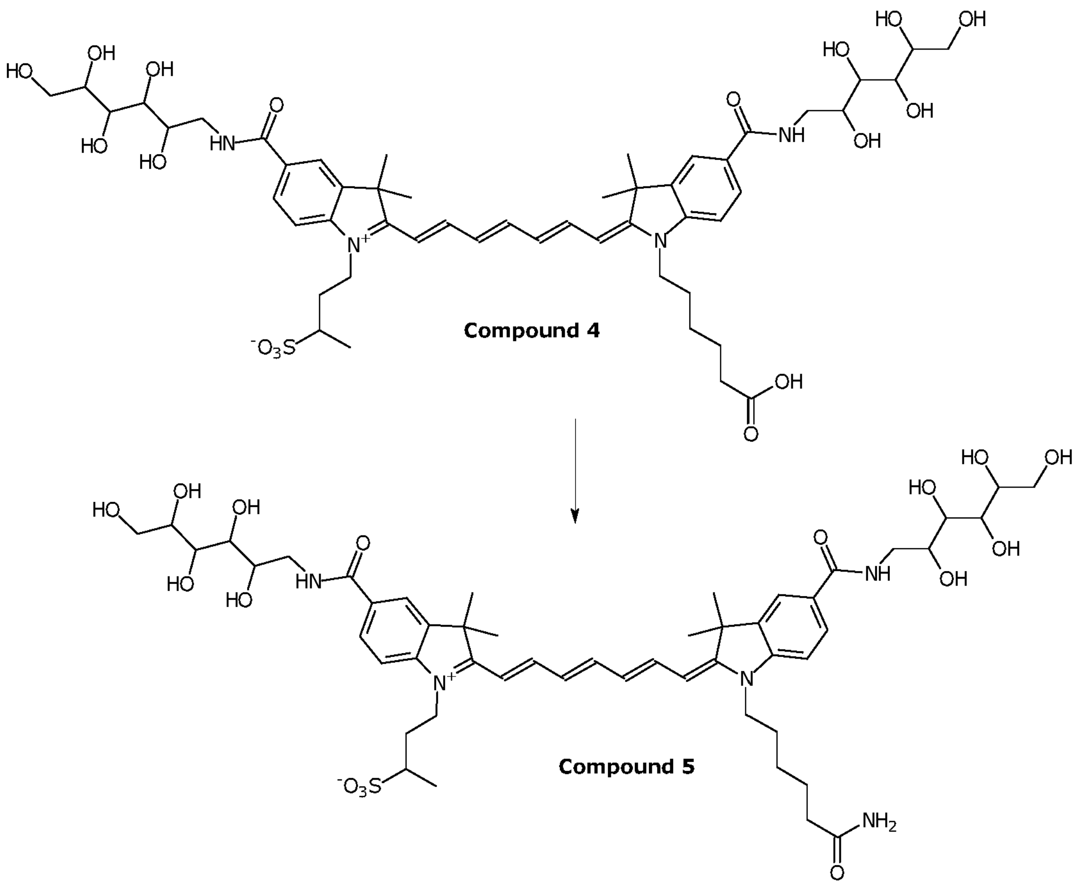

Compound 4

Compound 5

*Step f*

In a dried round bottom flask, N-methylmorpholine (10 μL, 0.091 mmol) was added to a solution of Compound 4 prepared as in example 1 (10 mg, 0.0096 mmol) in dry DMF (1 mL). After 30 minutes of stirring under nitrogen flow, a solution of HBTU (13 mg, 0.03 mmol) in DMF (1 mL) was added. After 1 h, a suspension of $NH_4Cl$ (10 mg, 0.19 mmol) in DMF (1 mL) was added. The mixture was kept under stirring for 2 days, then it was dried under vacuum and purified on a pre-packed C18 silica column with a water-acetonitrile gradient. Fractions containing pure product were combined, concentrated and freeze-dried, obtaining a blue solid (2,56 mg, 20% yield). HPLC purity at 756 nm 97.5%, MS: $[M + H]^+$ 1043.1.

**Example 3: Synthesis of compound 7**

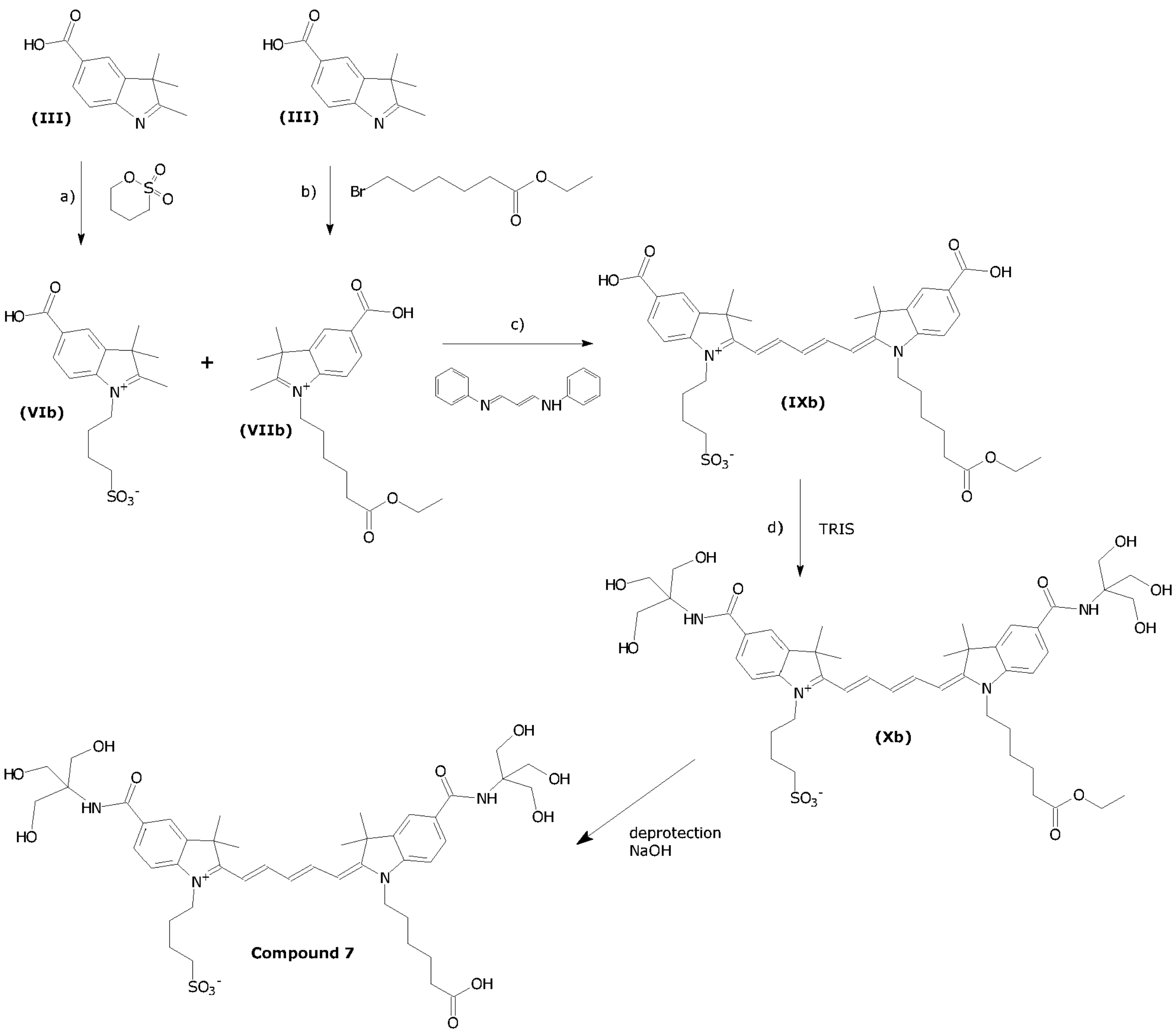


(III) (III) (VIb) (VIIb) (IXb) (Xb) Compound 7

*Preparation of intermediate Xb, Step J*

Intermediate IXb was prepared according to the procedure of Example 1, but using the reagent malonaldehyde dianilide hydrochloride for step c). Intermediate IXb (58 mg, 0.081 mmol) was suspended in dry DMF (10 mL). Trometamol (23.5 mg, 0.194 mmol), DIPEA (61 μL, 0.352 mmol) and HATU (73.77 mg, 0.194 mmol) were added. The reaction was stirred under inert atmosphere at RT for 2 hours. The solvent was distilled under reduced pressure and the crude was purified by flash chromatography on pre-packed C18 silica column with a water/methanol gradient. Fractions containing pure product were combined and distilled under vacuum and freeze-dried, giving a bright blue solid (60 mg, 80% yield). HPLC purity at 655 nm: 99.6%. MS: $[M + H]^+$ 927.3.

*Synthesis of compound 7, Step e*

Intermediate Xb (110 mg. 0.106 mmol) was dissolved in ethanol (2 mL) and water (18 mL) was added. The solution was heated at 40°C and pH was kept 11 with 0.1N NaOH, until the hydrolysis was completed (ca. 6 hours). PH was brought to 7 with 0.1N HCl and the crude was purified by flash chromatography on pre-packed C18 silica column with a water/methanol gradient. Fractions containing pure product were combined, distilled under vacuum and freeze-dried, giving a bright blue solid (18.7 mg, 19% yield). HPLC purity at 655 nm: 95.7%. MS: $[M + H]^+$ 899.3.

## Example 4: Synthesis of compound 8

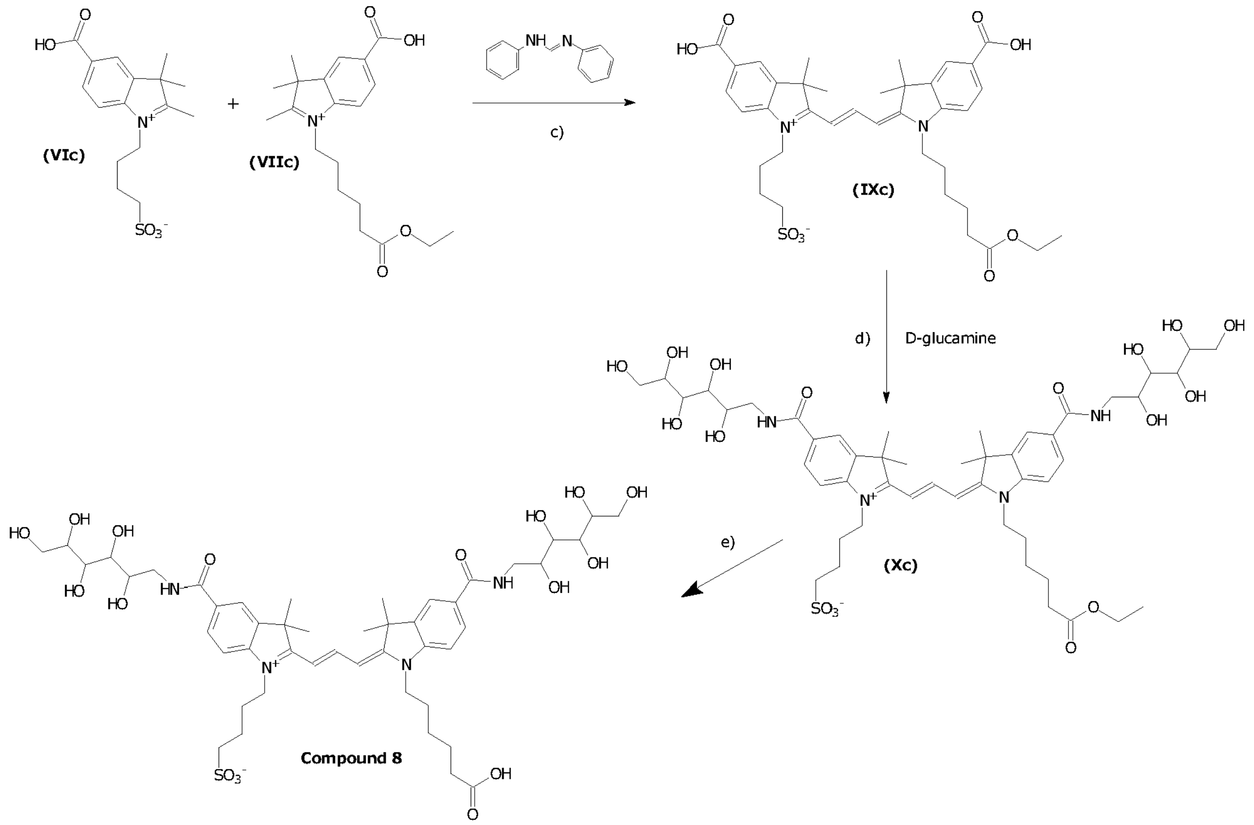


(VIc) (VIIc) (IXc) (Xc) Compound 8

Intermediates VIc and VIIc were prepared according to the procedure above described for intermediates VIa and VIIa. Analogously to Example 1, they were treated with the corresponding reagent N,N-diphenyl-formamidine to obtain the trimethine intermediate IXc.

*Preparation of intermediate Xc, Step J*

Intermediate IXc (62 mg, 0.089 mmol) was suspended in dry DMF (15 mL). D-Glucamine (33.9 mg, 0.187 mmol), DIPEA (62 μL, 0.356 mmol) and HATU (71.10 mg, 0.187 mmol) were added and the reaction was stirred under inert atmosphere at RT for 4 hours. The solvent was distilled under reduced pressure and the crude was purified by flash chromatography on pre-packed C18 silica column with a water/acetonitrile gradient. Fractions containing pure product were combined, distilled under vacuum and freeze-dried, giving a bright pink solid (82.4 mg, 91% yield). HPLC purity at 560 nm: 99.8%. MS: $[M + H]^+$ 1021.3.

*Synthesis of compound 8, Step e*

Intermediate Xc (81.4 mg. 0.08 mmol) was dissolved in ethanol (2 mL) and water (18 mL) was added. The solution was stirred at RT and pH was kept 11 with 0.1N NaOH, until the hydrolysis was completed (ca. 46 hours).Then, pH was adjusted to 7 with 0.1 N HCl and the crude purified by flash chromatography on pre-packed C18 silica column with a water/acetonitrile gradient. Fractions containing pure product were combined, distilled under vacuum and freeze-dried, giving a bright pink solid (79 mg, 99.4% yield). HPLC purity at 560 nm: 100%. MS: $[M + H]^+$ 993.3.

**Example 5: Synthesis of compound 1**

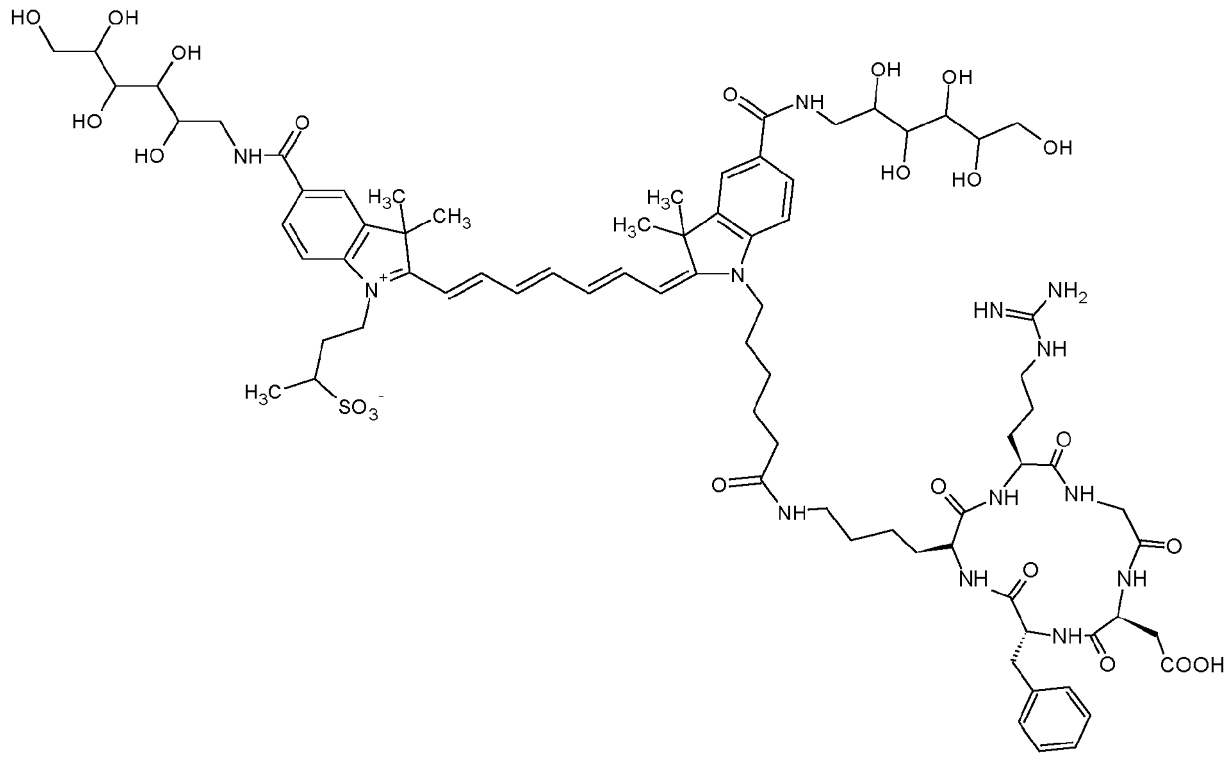

Compound 1 was syntesized by conjugation of the dye corresponding to Compound 4 and the cyclic pentapeptide c-RGDfK, wherein the conjugation has been carried out by two alternative procedures A or B. Compound 4 has been prepared as described in Example 1. The peptide c-RGDfK representing the targeting moiety is a commercial reagent.

*Procedure A - Conjugation step via direct coupling*

In a dried round bottom flask, N-methylmorpholine (10 μl, 0.09 mmol) was added to a solution of Compound 4, prepared as described in Example 1, (10 mg, 0.009 mmol) in dry DMF (1 mL). After 30 minutes of stirring under nitrogen flow, a solution of TBTU (6 mg, 0.02 mmol) in DMF (1 mL) was added. After 1 hour, a solution of c(RGD)fK (6 mg, 0.009 mmol) in DMF (1 mL) is added. The mixture was stirred at RT for 20 hours, then other TBTU (4 mg) and N-methylmorpholine (2 μL) were added, and after 2 hours c(RGD)fK (2 mg) was added. The mixture was stirred for additional 6 h, then it was concentrated and the crude was purified on analytical HPLC on C18 silica column with 0.1% ammonium acetate-acetonitrile gradient. Fractions containing the pure product were collected, concentrated and freeze-dried three times, to give a blue solid (4.4 mg, 29% yield). HPLC purity at 756 nm 99%, MS: $[M/2]^+$ 841.0.

*Procedure B - Conjugation step via previous activation with NHS ester*

Compound 4, prepared as described in Example 1, (4 mg, 0.0038 mmol) was suspended in dry DMF (3 mL) under nitrogen flow. N-methylmorpholine (0.8 μl, 0.0076 mmol) and TSTU (2.3 mg, 0.0076 mmol) were added and the solution was stirred at RT for 20 h. The desired product was precipitated by addition of cold diethyl ether, centrifuged and washed twice with diethyl ether.

The solid was dissolved in a solution of c(RGD)fK (3.0 mg, 0.0042 mmol) in borate buffer at pH 9 (2 mL). The solution was stirred overnight, then the pH was adjusted to 6 with 1N HCl and the crude was purified on analytical HPLC on C18 silica column with 0.1% ammonium acetate/acetonitrile gradient. Fractions containing the pure product were collected, concentrated and freeze-dried three times, to give a blue solid (5.2 mg, 83% yield). HPLC purity at 756 nm 99%, MS: $[M/2]^+$841.0.

**Example 6: Synthesis of compound 3**

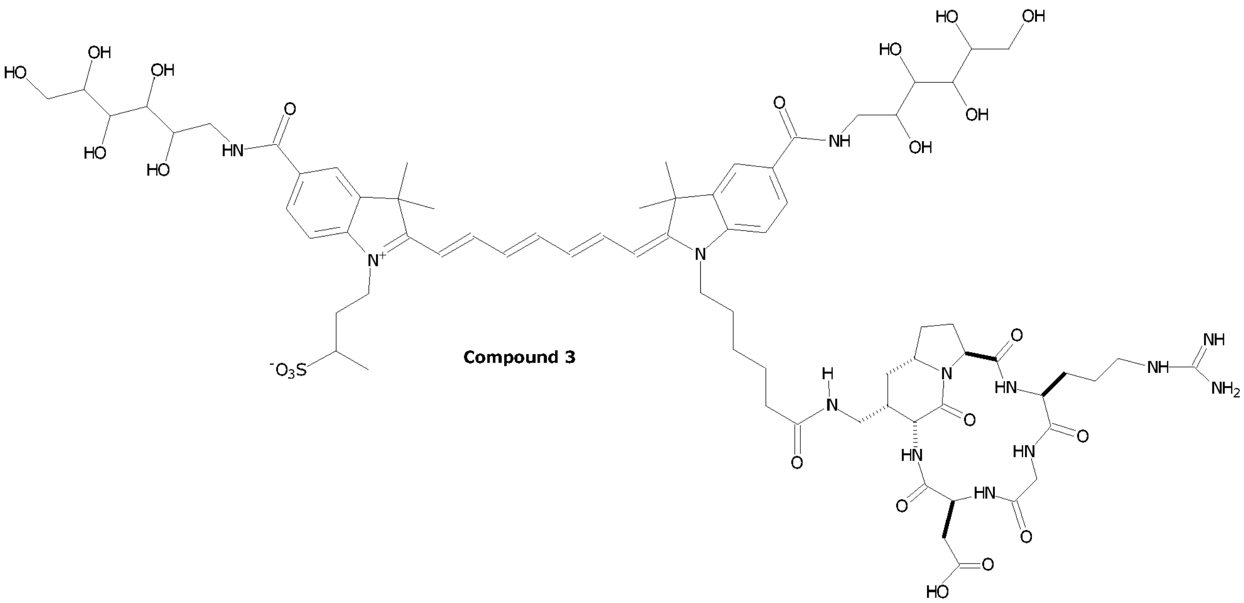

Compound 3

In a dried round bottom flask, N-methylmorpholine (10 μl, 0.09 mmol) was added to a solution of Compound 4 prepared as described in Example 1 (10 mg, 0.009 mmol) in dry DMF (1 mL). After 15 minutes of stirring under nitrogen flow, a solution of HBTU (3.6 mg, 0.01 mmol) in DMF (1 mL) was added. After 30 minutes, a solution of aza-cRGD-$NH_2$ (5.7 mg, 0.01 mmol) in DMF (1 mL) was added. The mixture was stirred for 2 days, then it was concentrated under vacuum and purified on analytic HPLC on C18 silica column with 0.1% ammonium acetate/acetonitrile gradient. Fractions containing the pure product were collected, concentrated and freeze-dried three times, to give a blue powder (3.57 mg, 21% yield). HPLC purity at 756 nm 99%, MS: $[M/2]^+$ 780.8.

**Example 7: Synthesis of compound 9**

The monoclonal antibody EGFR ligand Panitumumab (6 mg) was diluted up to 5 mg/mL in PBS and pH was adjusted by adding 120 μL of 1.0 M potassium phosphate pH 9. Compound 4-NHS ester (prepared as described in Example 5, Procedure B) was dissolved in DMSO at a concentration of 10 mg/ml; then the dye and the antibody were immediately mixed at a molar ratio of 2.5:1 and kept at room temperature in the dark for 3 h. After 3 h, the conjugation reaction mixture was layered onto phosphate buffered saline (PBS)-equilibrated Zeba Spin columns and centrifuged at 1500g for 2 min to separate the conjugate from the free dye. After filtration through a 0.22-μm polyethersulfone (PES) membrane, the conjugated Panitumumab solution in PBS at pH 7.4 was analyzed by SE-HPLC, RP-HPLC, UV/VIS spectrophotometry to determine concentration and purity. The molar conjugation ratio (dyes molecules coupled per antibody) was about 1.3.

**Example 8: Synthesis of compound 10**

The monoclonal antibody EGFR ligand Cetuximab (5 mg/mL) was dialyzed against PBS in a 10K Da MWCO membrane. 10 mg of dialyzed MAb (4.52 mg/mL, 68.8 nmol) were added to 221 μL of 1 M phosphate buffer pH 9. Compound 4-NHS ester (prepared as described in Example 5, Procedure B) was dissolved in DMSO at a concentration of 6.86 mM. 43.3 μL of dye were added to the Cetuximab solution (molar ratio 4.3:1) and kept at room temperature in the dark for 3 h. After this time, the conjugation reaction mixture was layered onto phosphate buffered saline (PBS)-equilibrated Zeba Spin column and centrifuged at 1000g for 2 min to separate the conjugate from the free dye. After filtration through a 0.22 μm polyethersulfone (PES) membrane, the conjugated Cetuximab solution in PBS at pH 7.4 was analyzed by SE-HPLC, RP-HPLC, UV/VIS spectrophotometry to determine concentration and purity. The molar conjugation ratio (dyes molecules coupled per antibody) was about 1.1.

**Example 9: Synthesis of compound 11**

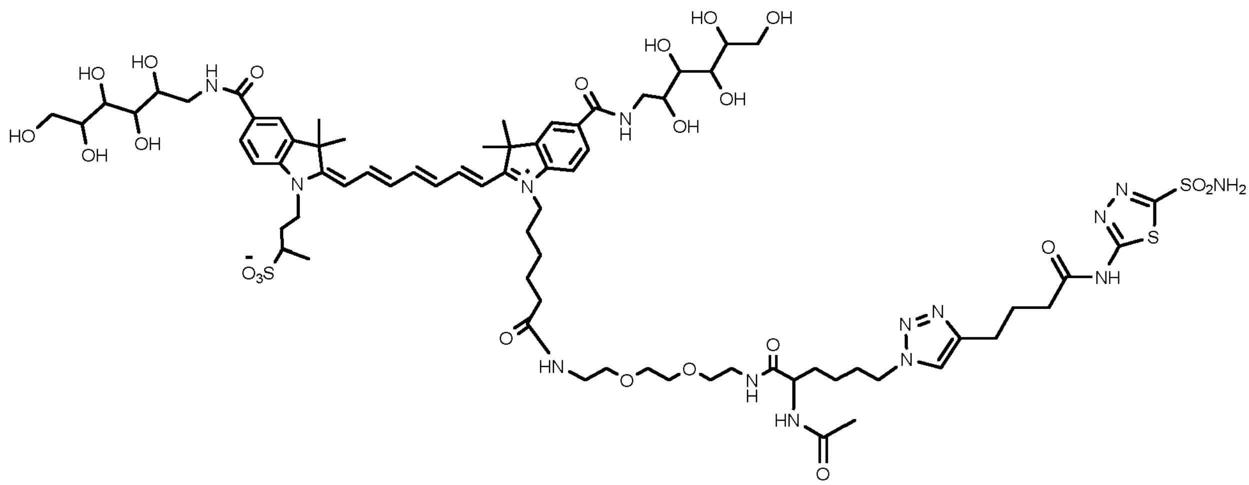

The small molecule CAIX ligand 4a, described in Wichert et al., Nat Chem 2015, 7, 241-249, was prepared according to the procedure therein disclosed and conjugated to compound 4. 11 mg of compound 4 (10,0 μmol) were dissolved in 1 mL of DMF, then 5.5 mg of PyBOP (10,0 μmol) and 7 μL of DIPEA (40,0 μmol) were added under continuous stirring. After 20 minutes, 9 mg of small molecule 4a (15,0 μmol) were dissolved in 1 mL of DMF and added to the reaction mixture which was stirred for additional 30 minutes at room temperature. Purification was performed by preparative HPLC with a yield of 60%. The isolated pure product was characterized by HPLC-UV-VIS-MS-ESI (+) using a Waters Atlantis dC18 column (μm, 4.6 × 150 mm). HPLC purity at 758 nm: 98%; MS: $[M/2]^+$ 823.3.

**Example 10: Synthesis of compound 12**

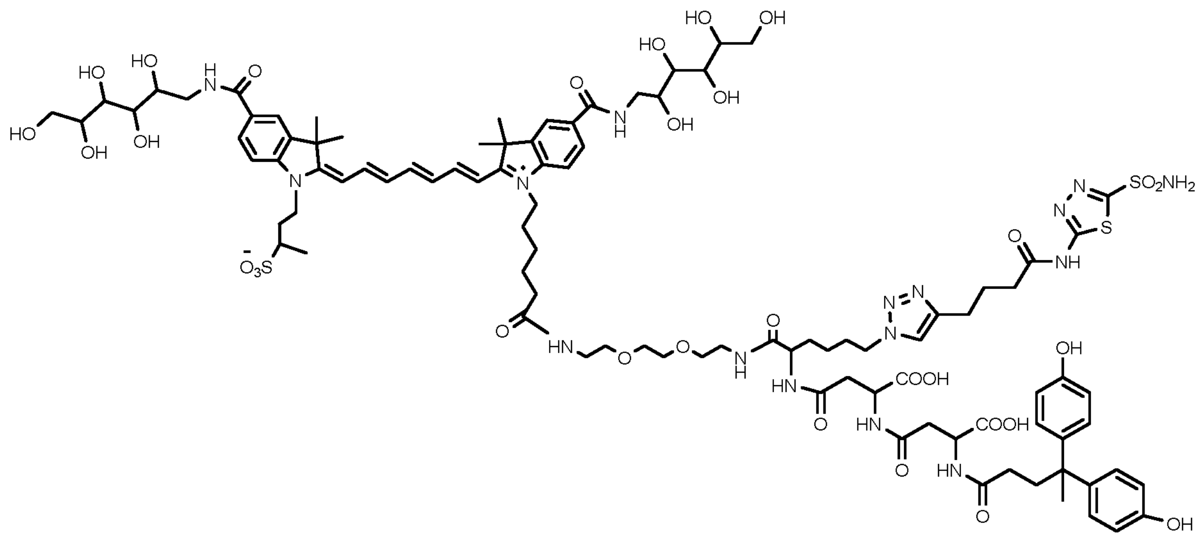

The small molecule CAIX ligand 8a, described in Wichert et al., Nat Chem 2015, 7, 241-249, was prepared according to the procedure therein disclosed and conjugated to Compound 4. 15 mg of Compound 4 (14.0 μmol) were dissolved in 1 mL of DMF, then 7.3 mg of PyBOP (14,0 μmol) and 10 μL of DIPEA (57,0 μmol) were added under continuous stirring. After 20 minutes, 22 mg of the small molecule 8a (21.0 μmol) were dissolved in 1 mL of DMF and added to the reaction mixture which was stirred for additional 30 minutes at RT. The purification was performed by preparative HPLC with a yield of 34%. The isolated pure product was characterized by HPLC-UV-VIS-MS-ESI (+) using a Waters Atlantis dC18 column (μm, 4.6 × 150 mm).

HPLC purity at 758 nm: 95%; MS: $[M/2]^+$ 1051.8.

**Example 11: Stability of intermediate (Xa)**

The stability of intermediate (Xa), prepared as described in Example 1 step d), has been evaluated in different conditions in order to perform the hydrolysis of the ethyl ester in the optimal conditions. In fact, it is known that such type of cyanines are not considerably stable in acidic and basic media. Contrary to other analogues bearing poly-sulfonyl groups, such as for instance compound DA364 described in WO2018/189136, being more stable in acidic conditions, intermediate (Xa) unexpectedly showed a remarkable stability when performing the hydrolysis in strong conditions, such as at pH 11 and 45°C for several hours, without degradation of the R1 and R2 groups.

Surprisingly, good results were also obtained performing the hydrolysis with enzymatic porcine and rabbit liver extracts, working at 1 mg/mL, pH 8 and 38°C in PBS buffer. Complete conversion was obtained after 20-24 hours only, without any degradation.

**Example 12: Optical properties**

The compounds of the invention have been characterized in terms of their optical properties *in vitro* in aqueous medium (i.e., water/PBS pH 7.4) and in a clinical chemistry control serum (Seronorm, Sero SA), mimicking the chemical composition and optical properties of human serum. All dye or dye-conjugate solutions were freshly prepared.

In particular, the excitation and emission maxima and the absolute fluorescence quantum yield ($\Phi$) of representative compounds of formula (I) and corresponding dyes of formula (II) are shown in Table II in comparison with the commercial bi-sulfonated heptamethine dye Indocyanine Green (ICG, Sigma), bi-sulfonated pentamethine dye sulfo-Cy5 (Lumiprobe) and bi-sulfonated trimethine dye sulfo-Cy3 (Lumiprobe).

*Table II - Excitation/Emission maxima and absolute fluorescence quantum yields of compounds of formula (I) (compounds 1 and 3) and (II) (compounds 4, 6, 7, 8)*

| **Cy7** | **Max Ex/Em (nm, PBS pH 7.4)** | **$\Phi$ (PBS pH 7.4)** | **$\Phi$ (Seronorm)** |
|---|---|---|---|
| ICG (Reference) | 780/810 | 1.5% | 7.8% |
| Compound 1 | 757/786 | 9.8% | 13.0% |
| Compound 3 | 757/785 | 10.6% | 11.7% |
| Compound 4 | 755/785 | 8.9% | 13.4% |
| **Cy5** | **Max Ex/Em (nm, PBS pH 7.4)** | **$\Phi$ (PBS pH 7.4)** | **$\Phi$ (Seronorm)** |
| Sulfo-Cy5 (Reference) | 645/665 | 27.0% | 35.6% |
| Compound 6 | 656/675 | 35.6% | 35.9% |
| Compound 7 | 655/675 | 35.1% | 35.1% |
| **Cy3** | **Max Ex/Em (nm, PBS pH 7.4)** | **$\Phi$ (PBS pH 7.4)** | **$\Phi$ (Seronorm)** |
| Sulfo-Cy3 (Reference) | 548/565 | 6.8% | n/a |
| Compound 8 | 557/573 | 14.5% | 20.0% |
| *n/a, not available* | | | |

The compounds of the invention are characterized by absorption maxima comprised in the range from about 550 nm to 760 nm. The fluorescence quantum yields obtained for the compounds of the invention was remarkably higher than the reference compound, with values from about 1.5 to 7 times greater than the corresponding reference compounds with the same length of polymethine chain. Remarkably, the compounds of the invention displayed greater quantum yield in the acqueous buffer PBS than the reference compounds with similar solubility properties.

**Example 13: Affinity to human albumin**

An analysis of the binding affinity of the compounds of the invention to human albumin was carried out and the results compared with the reference Indocyanine Green (ICG).

Binding affinity to human serum albumin (HSA; Sigma Aldrich, A9511) was measured using two methods, according to the level of binding affinity of the compounds.

The first method, optimal for compounds which strongly interact with HSA, is based on the analysis of the absorbance spectrum peak shift after the incubation of the dye in solutions containing HSA. Briefly, the samples were incubated at a fixed concentration (1 μM) with HSA dilutions ($1\times10^{-6}$ - $4\times10^{-4}$ M), in phosphate buffer for 5 min in the spectrophotometer at 25°C before measurements. The measure was performed at the maximum absorbance wavelength of the shifted peak.

The second method, optimal for compound with low affinity for HSA, is based on measuring the variation of the absorbance of solutions containing the dye and various concentrations of HSA after ultrafiltration. Briefly, each compound was incubated at a fixed concentration (2 μM) with HSA dilutions ($1\times10^{-6}$ - $4\times10^{-4}$ M), in phosphate buffer. The samples were centrifuged (10,000 g for 30 min at 25°C) in a Microcon device (10 kDa MWCO, Amicon Ultra-0.5 Centrifugal Filter Unit with Ultracel-10 membrane, Millipore) and the absorbance measurements of the filtrates were obtained with the spectrophotometer at the maximum absorbance wavelength of the fluorophore.

For both methods, the affinity constant ($K_A$, $M^{-1}$) was calculated by fitting the raw data with the following formula:

$$\frac{\Delta A}{b} = \frac{\Delta\varepsilon \cdot K_{RL}[L] \cdot R_t}{K_{RL}[L] + 1}$$

wherein

$\Delta A/b$ = Absorbance measured (b = 1 cm)
$K_{RL}$ = $K_A$ calculated by regression analysis (curve fitting)
$\Delta\varepsilon \cdot Rt$ calculated by regression analysis (curve fitting)
[L] = Albumin concentration

In the first method, $\Delta A/b$ corresponds to the absorbance measured for each sample, whereas in the second method $\Delta A/b$ is obtained subtracting the absorbance of the control sample (dye without HSA) to the absorbance of each other sample.

Both methods have demonstrated to provide comparable results, as shown by parallel experiments conducted on the commercial cyanine dye IRDye 800CW carboxylate (LI-COR Inc., Lincoln, USA) using the first method (HSA $K_A$ = 215,000 $M^{-1}$) and the second method (HSA $K_A$ = 216,000 $M^{-1}$). However, the measurement of the affinity constant is more precise when the suitable method is used as a function of the affinity level of the compound.

The results of the binding affinity measured for representative compounds of the invention with one of the two methods are reported in Table III and compared with the results obtained for the clinically available ICG dye and for ICG-RGD and DA364 as reference compounds.

*Table III Binding affinity to human serum albumin (HSA)*

| **Compound** | **HSA affinity ($K_A$, $M^{-1}$)** |
|---|---|
| ICG (Ref. compound) | 347,000 |
| ICG-RGD (Ref. compound) | 219,000 |
| DA364 (Ref. compound) | 28,670 |
| Compound 1 | 6,290 |
| Compound 3 | 4,200 |
| Compound 4 | 5,110 |
| Compound 6 | <1,000 |
| Compound 7 | 3,460 |
| Compound 8 | 2,070 |

As shown in Table III, the dyes and dyes-conjugates of the invention display a remarkably low binding affinity to human albumin compared to the available ICG and to many other known cyanine compounds, with affinity constants of one or two orders of magnitude lower. This advantageous feature relates both to dyes of formula (II) (e.g. Compound 4) and to the corresponding dye when conjugated with a targeting moiety (e.g. Compounds 1 and 3), suggesting that

the conjugation of the dyes with a targeting moiety does not affect the affinity to human albumin.

Rather, the RGD conjugates of formula (I), such as representative Compounds 1 and 3, have unexpectedly displayed much lower affinity for human albumin than other dyes known in the art when conjugated with the RGD targeting moiety, such as ICG-RGD (HSA $K_A$ = 219,000 $M^{-1}$, Capozza et al., 2018) or DA364 (HSA $K_A$ = 28,670 $M^{-1}$, WO2016/097317), as reported in Table III. Moreover, the reference compound DA364, when analysed exactly in the same experimental conditions of the present compounds, displayed a higher HSA affinity, with affinity constant ($K_A$) of 110,000 $M^{-1}$.

**Example 14: Receptor binding affinity**

The binding affinity of the conjugates of formula (I) to a specific receptor was determined to assess whether the targeting efficacy of the molecular vector is preserved after the labeling with the dyes of the invention.

*Peptide/peptidomimetic molecule conjugates*

As example of peptide/peptidomimetic conjugates, the receptor affinity of representative integrin-binding conjugates was evaluated through calculation of their $IC_{50}$ (half maximal inhibitory concentration), using an enzyme-linked immunosorbent assay (ELISA), as previously reported (Kapp et al., Sci. Rep. 2017, 7, 39805). Briefly, 96-well ELISA plates were coated overnight at 4°C with the extracellular matrix (ECM) protein Vitronectin in carbonate buffer (15 mM $Na_2CO_3$, 35 mM $NaHCO_3$, pH 9.6). Each well was then washed with PBS-T-buffer (phosphate-buffered saline/Tween20, 137 mM NaCl, 2.7 mM KCl, 10 mM $Na_2HPO_4$, 2 mM $KH_2PO_4$, 0.01% Tween20, pH 7.4) and blocked for 1 h at RT with TS-B-buffer (Tris-saline/BSA buffer; 20 mM Tris-HCl, 150 mM NaCl, 1 mM $CaCl_2$, 1 mM $MgCl_2$, 1 mM $MnCl_2$, pH 7.5, 1% BSA). In the meantime, a dilution series of the compound and internal standard was prepared in an extra plate. After washing the assay plate three times with PBS-T, 50 μL of the dilution series were transferred to each well. 50 μL of a solution of human recombinant integrin $\alpha_v\beta_3$ (R&D Systems, 1 μg/mL) in TS-B-buffer was transferred to the wells and incubated for 1 h. The plate was washed three times with PBS-T buffer, and then primary antibody anti-$\alpha_v\beta_3$ was added to the plate. After incubation and washing three times with PBS-T, the secondary anti-IgG peroxidase-labeled antibody was added to the plate and incubated for 1 h. After washing the plate three times with PBS-T, the plate was developed by quick addition of 3,3',5,5'-tetrametylbenzidine (TMB) and incubated for 5 min in the dark. The reaction was stopped with 3 M $H_2SO_4$, and the absorbance was measured at 450 nm with a plate reader (Victor3, Perkin Elmer).

The $IC_{50}$ of the representative compounds 1 and 3 was tested in duplicate, and the resulting inhibition curves were analyzed using GraphPad Prism version 4.0 for Windows (GraphPad Software). The inflection point defines the $IC_{50}$ value. All experiments were conducted using c(RGDfK) as internal standard.

The tested molecular probes, either coupled to c(RGDfK) (i.e. Compound 1) or to c(RGD)aza (i.e. Compound 3), showed comparable affinity to the human $\alpha_v\beta_3$ receptor, and similar affinity to the unconjugated reference peptidomimetic c(RGDfK), as reported in Table IV.

*Table IV - Binding affinity to the human $\alpha_v\beta_3$ integrin receptor of compounds 1 and 3 compared to the peptidomimetic c(RGDfK).*

| **Compound** | **Receptor affinity ($IC_{50}$, nM)** |
|---|---|
| c(RGDfK) | 2.69 ± 0.70 |
| Compound 1 | 2.96 ± 0.49 |
| Compound 3 | 2.64 ± 0.35 |

*Small molecule conjugates*

As example of small molecules conjugates, the inhibitory activity of the representative conjugates towards the CAIX enzyme was evaluated using the commercially available colorimetric kit K473 (BioVision). The protocol was performed according to the manufacturer's instructions, and the inhibitory potency (half maximal inhibitory concentration, $IC_{50}$) of the conjugates was compared to that of the unconjugated acetazolamide. Briefly, the kit utilizes the esterase activity of an active carbonic anhydrase enzyme on an ester substrate which releases a chromogenic product. The released product is quantified using an absorbance microplate reader (absorbance: 405 nm). In the presence of a carbonic anydrase specific inhibitor, as for acetazolamide and the conjugates of formula (I), the enzyme loses its activity which results in decrease of absorbance. The small molecules conjugates of formula (I) showed high inhibitory potency towards the enzyme carbonyc anhydrase, with $IC_{50}$ values in the low nanomolar range comparable to that of the unconjugated acetaxolamide (acetazolamide: 3.7 nM; Compound 11: 6.5 nM; Compound 12: 1.2 nM).

*Protein molecule conjugates*

As example of protein molecule conjugates, the receptor affinity of representative EGFR-binding conjugates was evaluated using the AlphaLISA kit AL366H (Perkin Elmer). In this AlphaLISA assay, a biotinylated EGF binds to the Streptavidin-coated Alpha Donor beads, while EGFR-Fc is captured by Anti-Human IgG Fc-specific AlphaLISA Acceptor beads. When EGF is bound to EGFR, Donor beads and Acceptor beads come into close proximity. The excitation of the Donor beads provokes the release of singlet oxygen molecules that triggers a cascade of energy transfers in the Acceptor beads, resulting in a sharp peak of light emission at 615 nm. Experiments were conducted by comparing the affinity of the conjugate to the relative unlabeled molecular vector. The unlabeled antibody Trastuzumab, which does not bind the EGFR receptor, was used as negative control. Diluition series of the samples (Compound 9 and Compound 10, and the relative unlabeled molecular vectors Panitumumab and Cetuximab, respectively) were incubated for 30 minutes with the EGFR Acceptor beads, followed by incubation for 30 minutes with the biotynilated EGF. Thus, the Streptavidin-conjugated Donor beads were added to the plate and incubated for 30 minutes. The absorbance was measured at 615 nm using a plate reader (EnSight, Perkin Elmer). No specific binding was observed for the antibody Trastuzumab. Differently, specific receptor binding was observed for Compound 9 ($IC_{50}$, 0.2 nM) and Compound 10 ($IC_{50}$, 0.4 nM), comparable to the unlabeled parent antibodies Panitumumab ($IC_{50}$, 0.5 nM) and Cetuximab ($IC_{50}$, 0.4 nM), respectively. Unexpectedly, it was found that large molecules like Cetuximab and Panitumumab maintained the native antigen binding properties even after conjugation with the dyes of the invention.

Overall, these results demonstrate that the conjugation of either small molecules, peptide/peptidomimetic or protein moieties to a dye compound of formula (II) does not impair the binding affinity of the final compound (I) to the target.

**Example 15: Cell uptake**

The human melanoma cell line WM-266-4 (ATCC, CRL-1676) was used as *in vitro* model to assess the cell uptake of representative integrin-binding Compounds 1 and 3, based on the high expression of the integrin receptors, particularly $\alpha_v\beta_3$, on the membrane of these cells (Capasso et al., PlosOne 2014).

Adherent cells at about 70% confluence were incubated with the compounds 1 or 3 (1 $\mu$M) for 2h at 37°C (5% COz) in presence of Dulbecco's Modified Eagle's Medium (DMEM) supplemented with 10% FBS, 2 mM glutamine, 100 IU/mL penicillin and 100 $\mu$g/mL streptomycin. After two washing steps with PBS, cells were detached using 0.1 mM EDTA in PBS, centrifuged and suspended in buffer (PBS, 0.5% BSA, 0.1% $NaN_3$) for flow cytometry experiments. Fluorescence Activated Cell Sorting (FACS) was used to detect the fluorescence signal within the cells, as measure of cell uptake. Samples were excited with an Argon laser and the emission detected using a 670 nm longpass filter. Values of fluorescence intensity were obtained from the histogram statistic produced by the instrument software.

To assess the specificity of receptor-mediated cell uptake, experiments were performed by incubating the cells with the molecular probes in presence of high concentration (100 $\mu$M) of the unlabeled molecular vector c(RGDfK) as competitor. The residual internalization was calculated by considering the value of fluorescence intensity in absence of the competitor as 100%.

Furthermore, to assess the effect of biological fluids on the cell uptake, parallel experiments were performed incubating the cells with a compounds of the invention in presence of human serum from male AB plasma (Sigma Aldrich, H4522) or human albumin (Sigma Aldrich, A9511). The residual internalization was calculated by considering the value of fluorescence intensity in absence of the serum as 100%. Such uptake assessment also represents an indication of the percentage of compound which is sequestered by the plasma proteins, in particular albumin, when it diffuses through the vascular compartment before reaching the tissue of interest and the particular targeted receptor.

In Table V the cell uptake performance of representative Compounds 1 and 3 of the invention is shown.

The present compounds displayed high cell uptake either in presence of human serum or in the presence of 4% HSA (Compound 1, residual uptake 70%), while they displayed a low residual uptake in the presence of the vector c(RGDfK) as competitor (Compound 1, residual uptake 10%; Compound 3, residual uptake 15%). Thus, for the present compounds it is observed that the internalization in the cells is receptor-mediated and is only slightly affected by the binding to human serum proteins, in particular albumin (about 10-20% of residual uptake), confirming the medium-to-low binding affinity to human albumin of the present compounds ($K_A$ = about 1-6 $\times$ $10^3$ $M^{-1}$, as shown in example 10).

Moreover, Table V reports a comparison of the residual cell uptake in presence of human serum for Compounds 1 and 3 with the reference compounds DA364, ICG-RGD (Capozza et al., Photoacoustic 2018, 11, 36-45) and ICG-c(RGDfK), prepared with the same method for ICG-RGD. These results show that the compounds of the present invention have been surprisingly found endowed with a higher efficacy in cell internalization with respect to similar compounds known in the art.

*Table V - Uptake of the integrin-binding fluorescent probes into WM-266-4 human melanoma cells in presence of human serum.*

| Compound | Residual cell uptake in presence of human serum |
|---|---|
| Cy5.5-cRGD (DA364) | 15% |
| ICG-cRGD | 12% |
| ICG-c(RGDfK) | 8% |
| Compound 1 | 80% |
| Compound 3 | 75% |

Notably, neither the interaction of the present compounds with the receptor on the cell surface, nor the internalization of the receptor-probe complex within the cell were impaired by the structure of the conjugated dyes, and particularly by the presence in position R1 and R2 of the compounds of moieties strongly hydrophilic and with high steric hindrance. Thus, the presence of the hydrophilic moieties on the conjugated dyes provide highly efficient and specific receptor binding and probe internalization even in presence of plasma proteins and albumin, which would sequester a conjugate lacking the hydrophilic moieties and negatively affect the binding efficiency.

**Example 16: Biodistribution and elimination**

Biodistribution and elimination of the fluorescent agents of the invention was evaluated by optical imaging after intravenous administration in male Balb/c nu/nu mice, 6-10 weeks of age (Charles River Laboratories). Briefly, mice housed 4 per cage and fed with VRF1 (P) sterile diet (Special Diets Services Ltd) up to the end of the acclimation period (5 days). Then, AIN-76a rodent diet irradiated (Research Diets), a special diet that reduces auto-fluorescence, was used up to the end of the experiments. Imaging experiments were performed using the preclinical optical system IVIS Spectrum (Perkin Elmer).

*In vivo* imaging was performed under gas anesthesia (Sevofluorane 6-8% in oxygen). Animals were intravenously injected with the compounds and imaged longitudinally at 30 min, 1h, 2h, 3h, 4h, 6h, and 24h post-administration. Thus, animals were euthanized by anesthesia overdose, and the main tissues and organs of interest were excised for *ex vivo* optical imaging. Regions of interest (ROIs) were drawn on the tissues of interest for each fluorescence image at every time point to evaluate signal intensity (expressed as Average Radiant Efficiency). The ratio between the fluorescence signal in the muscle (background tissue) and excretory organs (kidney, liver) was then calculated to assess the contrast. The tissue half-life was calculated by fitting the fluorescence signal decay in the muscle with a bi-exponential decay equation model using GraphPad Prism Software (version 5 for Windows).

The tissue kinetics of Compound 1 is shown in Figure 1, where the fluorescence signal decay in the muscle tissue was plotted vs. time to assess the washout of the product at different doses. Remarkably, Compound 1 showed a very favorable elimination kinetics with fast tissue washout and improved body elimination and, particularly it displayed very short (< 1 hour) tissue half-lives at all doses (0.3, 1, 3 nmol/mouse) tested. The compound quickly distributed in the body and accumulated in the bladder, suggesting renal elimination. Moreover, it displayed fast body elimination and low retention, advantageously allowing for early imaging after administration.

Table VI shows the ratios between the fluorescence signal detected by optical imaging in the excised excretory organs (liver and kidney) vs. the muscle, 24 hours after the intravenous (iv) administration of 1 nmol/mouse of Compound 1 or 3.

*Ex vivo* imaging of excised organs and tissues revealed very low retention of Compounds 1 and 3 in the excretory organs kidney and liver, associated with the faster body elimination and reduced non-specific accumulation.

*Table VI - Ratio between the fluorescence signal decay in excretory organs and in the muscle 24 hours after iv administration.*

| Compound | Kidney to muscle ratio | Liver to muscle ratio |
|---|---|---|
| Compound 1 | $3.18 \pm 0.32$ | $2.92 \pm 0.21$ |
| Compound 3 | $2.90 \pm 0.55$ | $2.50 \pm 0.32$ |

## Example 17: Tumor uptake

The tumor uptake of the compounds of the present invention has been assessed by optical imaging after intravenous administration in mice.

Tumor uptake experiments were carried out in an animal model of human glioblastoma (subcutanous) overexpressing the integrin receptors, particularly $\alpha_v\beta_3$. Briefly, human glioblastoma U87MG cells (ATCC, HTB-14) were cultured in Eagle's Minimum Essential Medium (EMEM) supplemented with 10% Fetal Bovine Serum (FBS), 2 mM glutamine, 100 IU/mL penicillin, 100 $\mu$g/mL streptomycin. Male Balb/c nu/nu mice, 4-6 weeks of age (Charles River Laboratories), underwent subcutaneous implantation (right flank) of about 10 million cells suspended in 0.1 mL of EMEM. Mice were housed 4 per cage with food and water ad libitum. Animals were fed with VRF1 (P) sterile diet (Special Diets Services Ltd) up to the end of the acclimation period (5 days). Then, AIN-76a rodent diet irradiated (Research Diets), a special diet that reduces auto-fluorescence, was used up to the end of the experiments. Tumor growth was monitored by longitudinal assessments using a caliper up to the target size of 300-600 $mm^3$ (3-4 weeks after cell implantation). Imaging experiments were performed using the preclinical optical system IVIS Spectrum (Perkin Elmer).

*In vivo* imaging was performed under gas anesthesia (Sevofluorane 6-8% in oxygen). Animals were intravenously injected (lateral tail vein) with the compounds, and imaged longitudinally at 30 min, 1h, 2h, 3h, 4h, and 24h post-administration. Regions of interest (ROIs) were drawn on the tissues of interest for each fluorescence image at every time point to evaluate signal intensity (expressed as Average Radiant Efficiency). The ratio between the fluorescence signal in the tumor and in the muscle (background tissue) was then calculated to assess the contrast.

The fluorescence decay values of representative Compound 1 in the tumor and muscle tissue and the tumor-to-backgroud ratio are displayed in Figure 2. These results show a remarkably high tumor uptake for the representative Compound 1 already at early time-points (1-2 hours after administration), providing a very high tumor-to-background contrast (TBR ~2-2.5) and low retention in the healthy tissue, suggesting tumor-specific accumulation.

In fact, the present compounds are quickly eliminated from the body, particularly from the healthy tissues (muscle), while a considerable target-mediated accumulation can be observed for the conjugated dyes of formula (I) in the targeted tissues.

Parallel experiments were performed in an animal model of human head and neck cancer (orthotopic), using Detroit-562 cells, overexpressing in particular integrin receptor $\alpha_v\beta_6$. Briefly, the human pharyngeal carcinoma cells Detroit-562 (ATCC, CCL-138) were cultured in Eagle's Minimum Essential Medium (EMEM) supplemented with 10% Fetal Bovine Serum (FBS), 2 mM glutamine, 100 IU/mL penicillin, 100 $\mu$g/mL streptomycin. Male Balb/c nu/nu mice, 4-6 weeks of age (Charles River Laboratories), underwent orthotopic implantation in the anterior portion of the tongue of about 2.5 million cells suspended in 0.03 mL di EMEM. Mice were housed 4 per cage with food and water ad libitum. Animals were fed with VRF1 (P) sterile diet (Special Diets Services Ltd) up to the end of the acclimation period (5 days). Then, AIN-76a rodent diet irradiated (Research Diets), a special diet that reduces auto-fluorescence, was used up to the end of the experiments. Tumor growth was monitored by longitudinal assessments using a caliper up to the target size of 10-20 $mm^3$ (7-10 days after cell implantation).

Imaging experiments were performed using the preclinical optical system IVIS Spectrum (Perkin Elmer). Animals were intravenously injected (lateral tail vein) with 3 nmol/mouse, at 24 hours post-administration were euthanized by anesthesia overdose, and the tongues were excised for *ex vivo* optical imaging. Regions of interest (ROIs) were drawn on the anterior portion of the tongue (site of tumor cell implantation) and on the posterior region (healthy tissue) to derive the tumor-to-background ratio. One healthy animal (no tumor implantation) was administered with 3 nmol/mouse of the Compound 1 and imaged as described above to be used as negative control.

*Ex vivo* imaging performed 24 hours after the administration of the compound 1 revealed a bright region in the tongue site of implantation of the tumor cells. Differently, the healthy region in the back of the tongue showed low signal, similar to that detected in healthy mice, suggesting a low retention in healthy tissue. The administration of the compound of the invention reveals the location of the tumor with high tumor-to-background contrast (as shown in Figure 3).

Parallel experiments were performed in an animal model of human colorectal cancer (subcutaneous), using HT-29 cells, expressing low levels of integrin receptors. Briefly, the human colorectal adenocarcinoma cells HT-29 (ATCC, HTB-38) were cultured in McCoy's 5A medium supplemented with 10% foetal bovine serum, 2 mM glutamine, 100 IU/mL penicillin and 100 $\mu$g/mL streptomycin. Male Athymic nude mice, 4-6 weeks of age (Envigo), underwent subcutaneous implantation (right flank) of about 5 million cells suspended in 0.1 mL of serum-free medium. Mice were housed 4 per cage with food and water ad libitum. Animals were fed with VRF1 (P) sterile diet (Special Diets Services Ltd) up to the end of the acclimation period (5 days). Then, AIN-76a rodent diet irradiated (Research Diets), a special diet that reduces auto-fluorescence, was used up to the end of the experiments. Tumor growth was monitored by longitudinal assessments using a caliper up to the target size of 300-600 $mm^3$ (3-4 weeks after cell implantation). Imaging experiments were performed using the preclinical optical system IVIS Spectrum (Perkin Elmer).

*In vivo* imaging was performed under gas anesthesia (Sevofluorane 6-8% in oxygen). Animals were intravenously injected (lateral tail vein) with the compounds of interest, and imaged longitudinally at 30 min, 1h, 2h, 3h, 4h, 6h,

and 24h post-administration. Regions of interest (ROIs) were drawn on the tissues of interest for each fluorescence image at every time point to evaluate signal intensity (expressed as Average Radiant Efficiency). The ratio between the fluorescence signal in the tumor and in the muscle (background tissue) was then calculated to assess the contrast.

The fluorescence decay values in the tumor and muscle tissue and the tumor-to-backgroud ratio are displayed in Figure 4 for Compound 1.

These results show high tumor uptake for the representative Compound 1 already at early time-points and fast clearance from the healthy tissues, resulting in a moderate tumor-to-background ratio (TBR ~1.2-1.5) sufficient to clearly delineate the tumor tissue from the healthy background.

The target specificity of the representative Compound 1 was compared to that of the reference compound DA364 in an animal model of human cancer which overexpress the human integrin receptor $\alpha V\beta_3$, as described above. Both compounds were administered at the dose of 1 nmol/mouse together either with (blocking group) or without (control group) an excess of unlabeled cRGD peptidomimetic (200 nmol/mouse). After 24 hours from injection, the mice were euthanized, the tumor tissues excised and imaged using the IVIS Spectrum fluorescence system (Perkin Elmer). The blocking protocol prompted a 10% reduction in fluorescence signal intensity (the imaging surrogate of uptake) in the tumor tissue of animals administered with DA364. Differently, the blocking protocol prompted a reduction of 75% in fluorescence in the tumors of animals that received Compound 1, denoting a higher *in vivo* receptor specificity for the compound of the invention (see Table VII).

*Table VII - Determination of the in vivo receptor specificity by blocking experiment.*

| Compound | Reduction of tumor fluorescence after receptor blocking |
|---|---|
| DA364 (Reference) | 10% (n=5/group) |
| Compound 1 | 75% (n=5/group) |

**References**

1. Onda N. et al., Int J Cancer, 2016, 139, 673-682
2. Stummer W. et al., Neurosurgery, 2015, 77(5), 663-673
3. Licha et al., Photochemistry and Photobiology, 2000, 72(3), 392-398
4. Tummers Q. et al., PlosOne 2015
5. Achilefu S. et al., J Med Chem, 2002, 45, 2003-2015
6. Bunschoten A. et al., Bioconjugate Chem., 2016, 27, 1253-1258
7. US 6,913,743 B2
8. WO2018/189136
9. WO2016/097317
10. Capozza et al., Photoacoustics, 2018, 11, 36-45
11. US 6,329,531 B1
12. Ebert et al., J. Biomed. Optics, 2011, 16(6), 066003
13. WO2005/123768
14. WO2012/088007
15. T.W. Green and P.G.M.Wuts, Protective Groups in Organic Synthesis, Wiley, N.Y. 2007, 4th Ed., Ch. 5
16. Kapp et al., Sci Rep, 2017, 7, 3905
17. Wichert et al., Nat Chem 2015, 7, 241-249
18. Li et al., FASEB J 2005, 19, 1978-85
19. Williams et al., Chem Biol Drug Des 2018, 91, 605-619
20. Mujumdar R.B. et al., Bioconjugate Chem. 1993, 4(2), 105-111
21. Yamana et al, Chem. Pharm. Bull., 1972, 20(5), 881-891


**Claims**

**1.** A compound of formula (I)

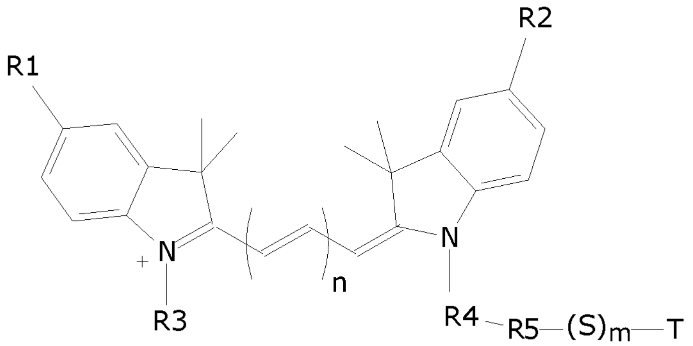 (I)

wherein

**R1** and **R2** are independently a -CO-NH-Y group, wherein Y is selected from a linear or branched alkyl, cycloalkyl and heterocyclyl, substituted by at least two hydroxyl groups;
**R3** is linear or branched alkyl substituted by a group selected from $-NH_2$, $-SO_3H$, -COOH and $-CONH_2$;
**R4** is linear or branched bivalent alkyl;
**R5** is selected from -COO- and -CONH-;
**S** is a spacer;
**T** is a targeting moiety;
**n** is an integer equal to 1, 2 or 3; and
**m** is an integer equal to 0 or 1.
or a pharmaceutically acceptable salt thereof.

**2.** The compound of formula (I) according to claim 1, wherein n is 3, R3 is alkyl substituted by $-SO_3H$, and R1 and R2 are independently selected from the group consisting of

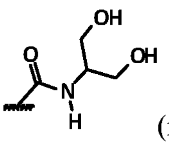 (iii), 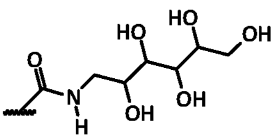 (iv), 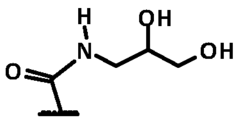 (v), 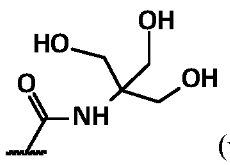 (vi),

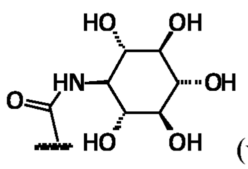 (vii) and 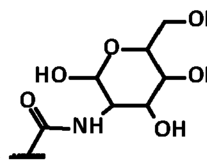 (viii).

**3.** The compound of formula (I) according to claim 1 or 2, which is represented by formula (Ia)

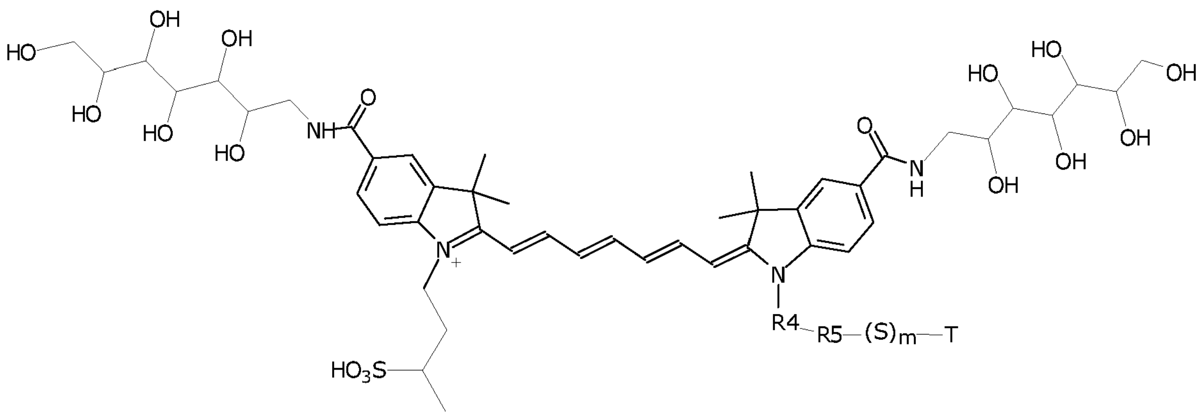

(Ia)

wherein R4, R5, S, m and T are as defined in claim 1.

**4.** The compound of formula (I) according to claims 1, 2 or 3, wherein S is selected from - $NH(CH_2)_pCOO$-, -$NH(CH_2CH_2O)_pCH_2CH_2COO$- and -$NH(CH_2CH_2O)_pCH_2CH_2NH$-, wherein p is an integer comprised between 0 and 20.

**5.** The compound of formula (I) according to any of the preceding claims, wherein T is targeting moiety selected from the group consisting of a small molecule, a protein, a peptide, a peptidomimetic, an enzyme substrate, an antibody or fragment thereof and an aptamer.

**6.** The compound of formula (I) according to any of the preceding claims, wherein T is a moiety interacting with an integrin receptor.

**7.** The compound of formula (I) according to any of the preceding claims for use as fluorescence contrast agent for the detection and demarcation of a tumor margin in guided surgery of an individual patient.

**8.** The compound of formula (I) for use according to claim 7 wherein the detection and demarcation of the tumor margin is carried out under NIR radiation.

**9.** The compound of formula (I) for use according to claim 8, wherein said tumor is a tumor selected from brain cancer, breast cancer, head and neck cancer, ovarian cancer, prostate cancer, esophageal cancer, skin cancer, gastric cancer, pancreatic cancer, bladder cancer, oral cancer, lung cancer, renal cancer, uterine cancer, thyroid cancer, liver cancer, and colorectal cancer.

**10.** A pharmaceutical diagnostic composition comprising a compound of formula (I) as defined in claim 1 and at least one pharmaceutically acceptable carrier or excipient.

**11.** Diagnostic kit comprising a compound of formula (I) as defined in claim 1 together with additional adjuvants thereof for implementing the optical imaging.

**12.** A compound of formula (I) according to claim 1 selected from

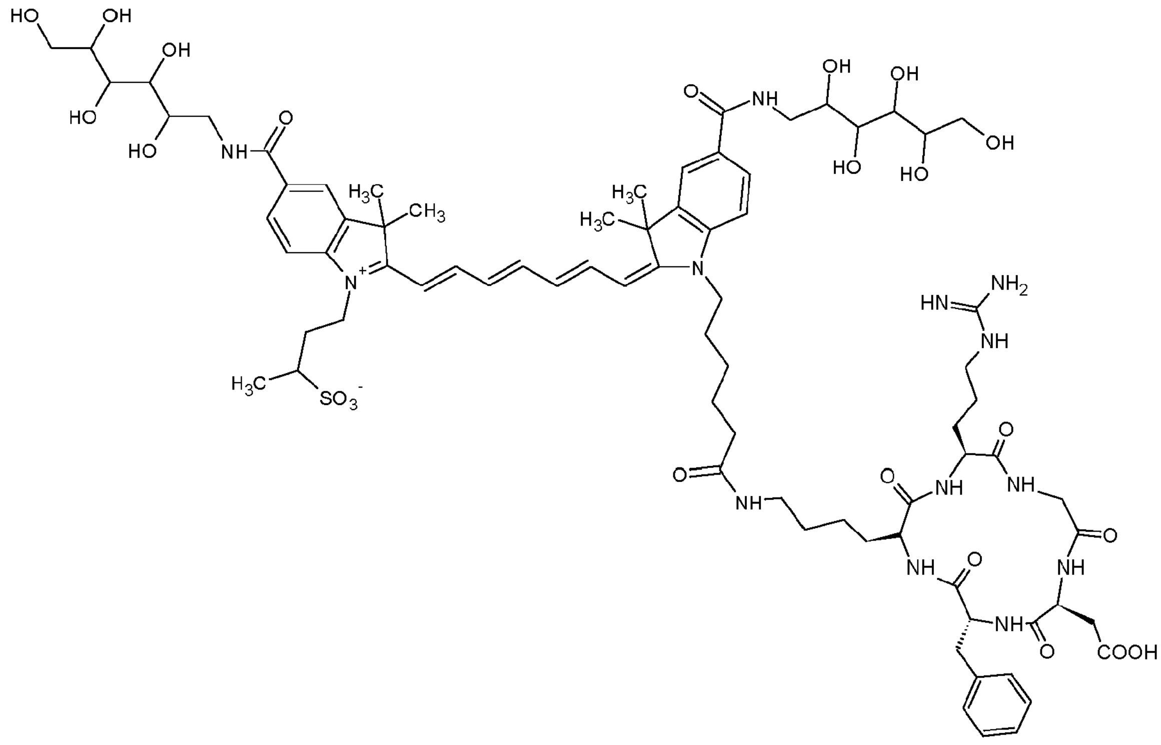

and

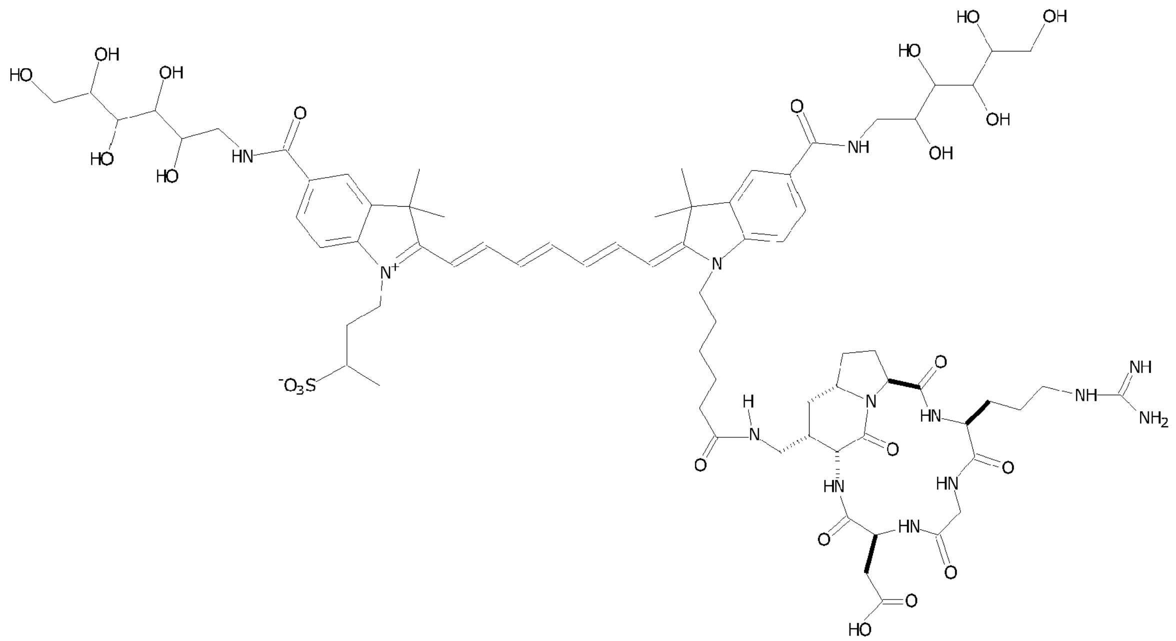

13. An intermediate compound of formula (II)

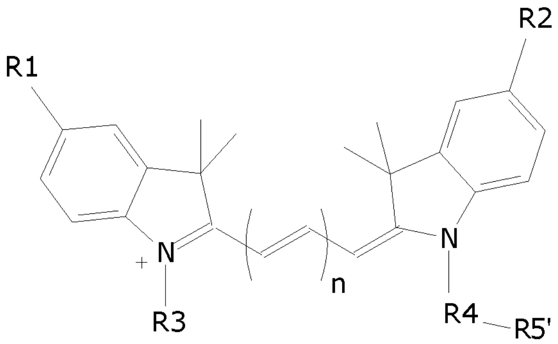
(II)

wherein

**R1, R2, R3, R4** and **n** are as defined in claim 1 and
**R5'** is selected from -COOH, $-CONH_2$, -COO-Pg and -CONH-Pg, wherein Pg is a protecting group, or a pharmaceutically acceptable salt thereof.

**14.** An intermediate of formula (II) according to claim 13, wherein n is 3, R3 is alkyl substituted by $-SO_3H$, and R1 and R2 are independently selected from the group consisting of

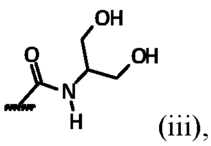 (iii), 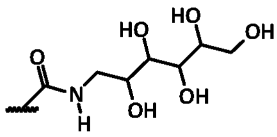 (iv), 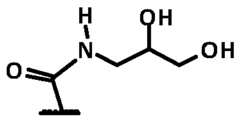 (v), 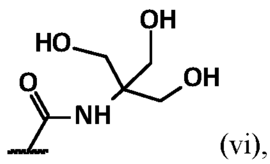 (vi),

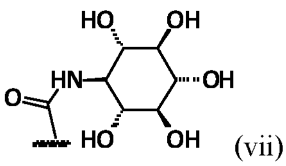 (vii) and 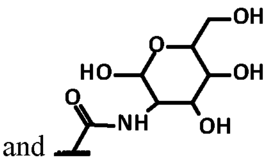 (viii).

**15.** An intermediate of formula (II) according to claims 13 or 14, which is represented by formula (IIa)

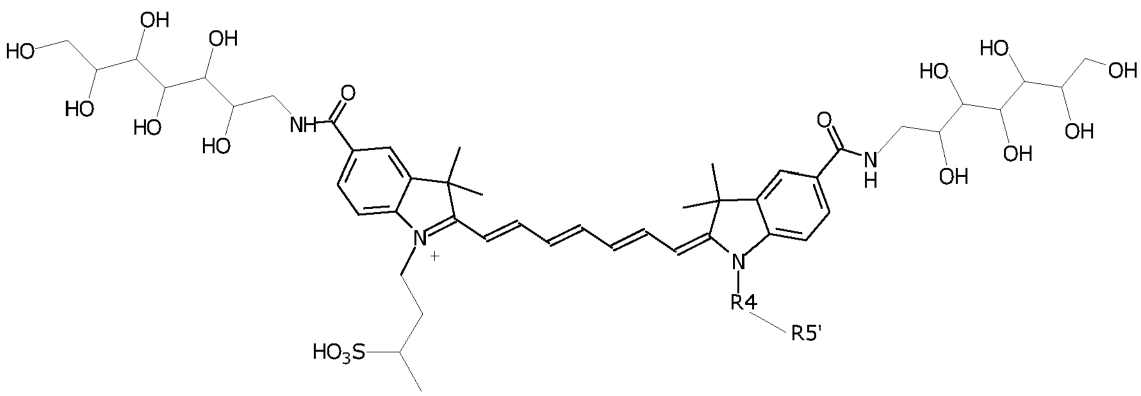

(IIa)

wherein R4 and R5' are as defined in claim 13.

## Patentansprüche

1. Verbindung der Formel (I)

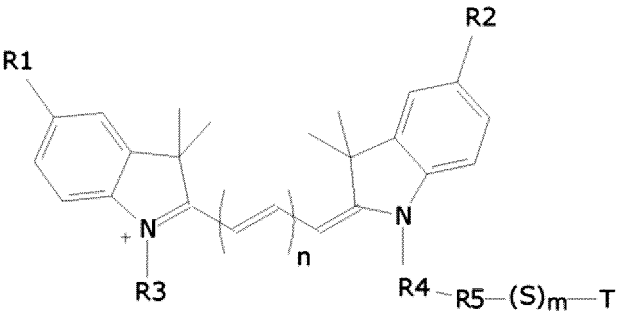

(I)

wobei

**R1** und **R2** unabhängig für eine -CO-NH-Y-Gruppe stehen, wobei Y aus einem linearen oder verzweigten Alkyl, Cycloalkyl und Heterocyclyl, das durch mindestens zwei Hydroxylgruppen substituiert ist, ausgewählt ist;
**R3** für lineares oder verzweigtes Alkyl, das durch eine aus $-NH_2$, $-SO_3H$, -COOH und $-CONH_2$ ausgewählte Gruppe substituiert ist, steht;
**R4** für lineares oder verzweigtes zweiwertiges Alkyl steht;
**R5** aus -COO- und -CONH- ausgewählt ist;
**S** für einen Spacer steht;
**T** für eine Zielsteuerungsgruppierung steht;
**n** für eine ganze Zahl mit einem Wert von 1, 2 oder 3 steht und
**m** für eine ganze Zahl mit einem Wert von 0 oder 1 steht;
oder ein pharmazeutisch unbedenkliches Salz davon.

2. Verbindung der Formel (I) nach Anspruch 1, wobei n für 3 steht, R3 für Alkyl, das durch $-SO_3H$ substituiert ist, steht und R1 und R2 unabhängig aus der Gruppe bestehend aus

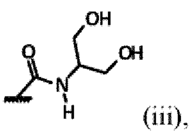 (iii), 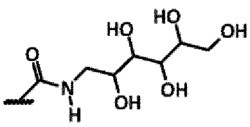 (iv), 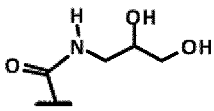 (v), 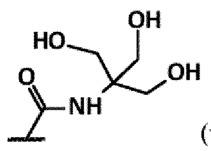 (vi),

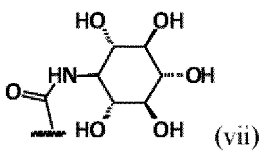 (vii) und 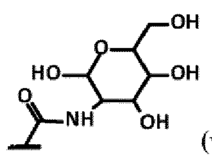 (viii)

ausgewählt sind.

3. Verbindung der Formel (I) nach Anspruch 1 oder 2, die durch Formel (Ia) wiedergegeben wird:

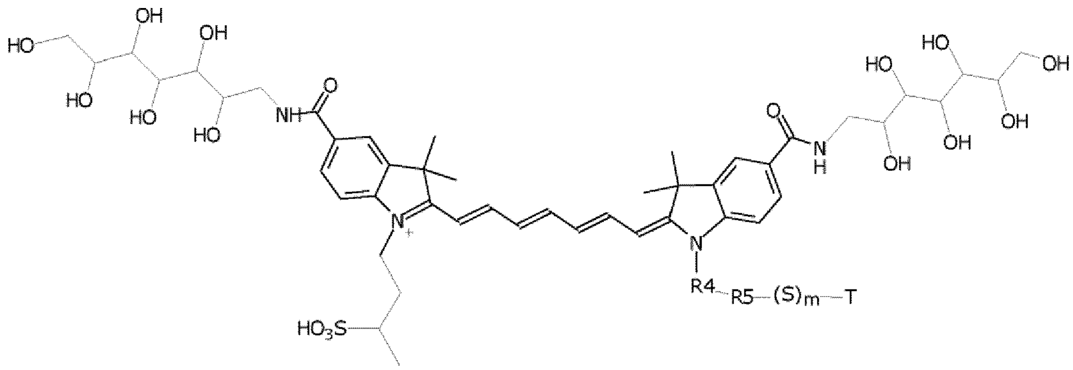

(Ia)

wobei R4, R5, S, m und T wie in Anspruch 1 definiert sind.

4. Verbindung der Formel (I) nach Anspruch 1, 2 oder 3, wobei S aus $-NH(CH_2)_pCOO-$, $-NH(CH_2CH_2O)_pCH_2CH_2COO-$ und $-NH(CH_2CH_2O)_pCH_2CH_2NH-$ ausgewählt ist, wobei p für eine ganze Zahl zwischen 0 und 20 steht.

5. Verbindung der Formel (I) nach einem der vorhergehenden Ansprüche, wobei T für eine Zielsteuerungsgruppierung steht, die aus der Gruppe bestehend aus einem kleinen Molekül, einem Protein, einem Peptid, einem Peptidomimetikum, einem Enzymsubstrat, einem Antikörper oder Fragment davon und einem Aptamer ausgewählt ist.

6. Verbindung der Formel (I) nach einem der vorhergehenden Ansprüche, wobei T für eine mit einem Integrinrezeptor wechselwirkende Einheit steht.

7. Verbindung der Formel (I) nach einem der vorhergehenden Ansprüche zur Verwendung als Fluoreszenzkontrastmittel zum Nachweis und zur Abgrenzung eines Tumorrandes bei einem geführten chirurgischen Eingriff bei einem individuellen Patienten.

8. Verbindung der Formel (I) zur Verwendung nach Anspruch 7, wobei der Nachweis und die Abgrenzung des Tumorrandes unter NIR-Strahlung erfolgen.

9. Verbindung der Formel (I) zur Verwendung nach Anspruch 8, wobei es sich bei dem Tumor um einen Tumor handelt, der aus Gehirnkrebs, Brustkrebs, Kopf- und Halskrebs, Eierstockkrebs, Prostatakrebs, Speiseröhrenkrebs, Hautkrebs, Magenkrebs, Bauchspeicheldrüsenkrebs, Blasenkrebs, Mundkrebs, Lungenkrebs, Nierenkrebs, Gebärmutterkrebs, Schilddrüsenkrebs, Leberkrebs und Kolorektalkrebs ausgewählt ist.

10. Pharmazeutische diagnostische Zusammensetzung, umfassend eine Verbindung der Formel (I) gemäß Anspruch 1 und mindestens einen pharmazeutisch unbedenklichen Träger oder Hilfsstoff.

11. Diagnostisches Kit, umfassend eine Verbindung der Formel (I) gemäß Anspruch 1 zusammen mit zusätzlichen Adjuvantien davon zur Durchführung der optischen Bildgebung.

12. Verbindung der Formel (I) nach Anspruch 1, ausgewählt aus

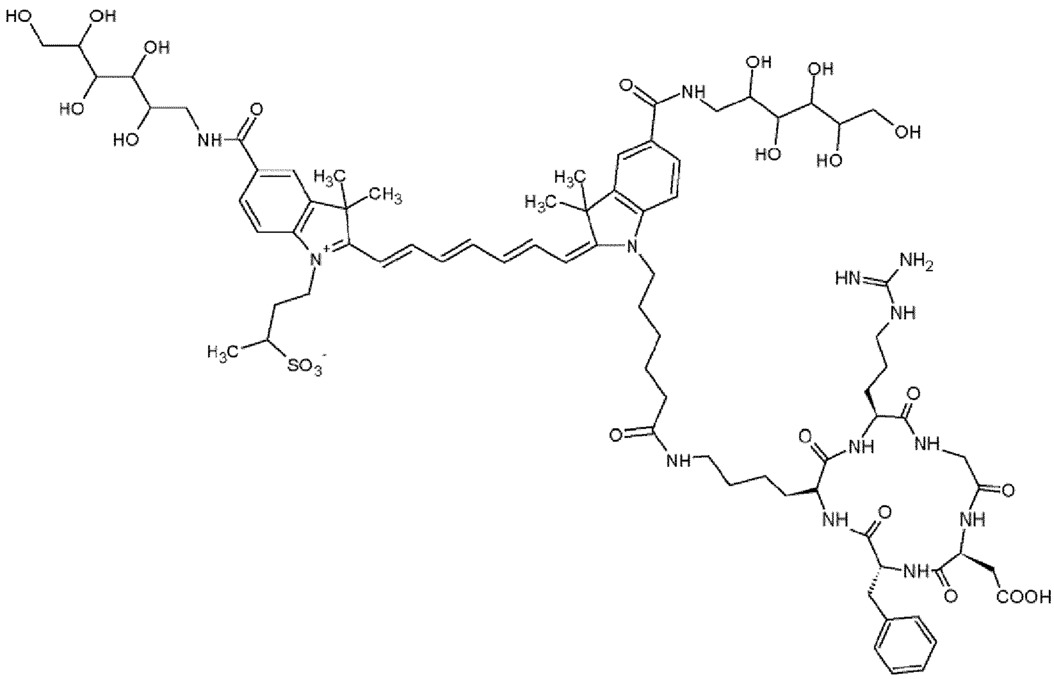

und

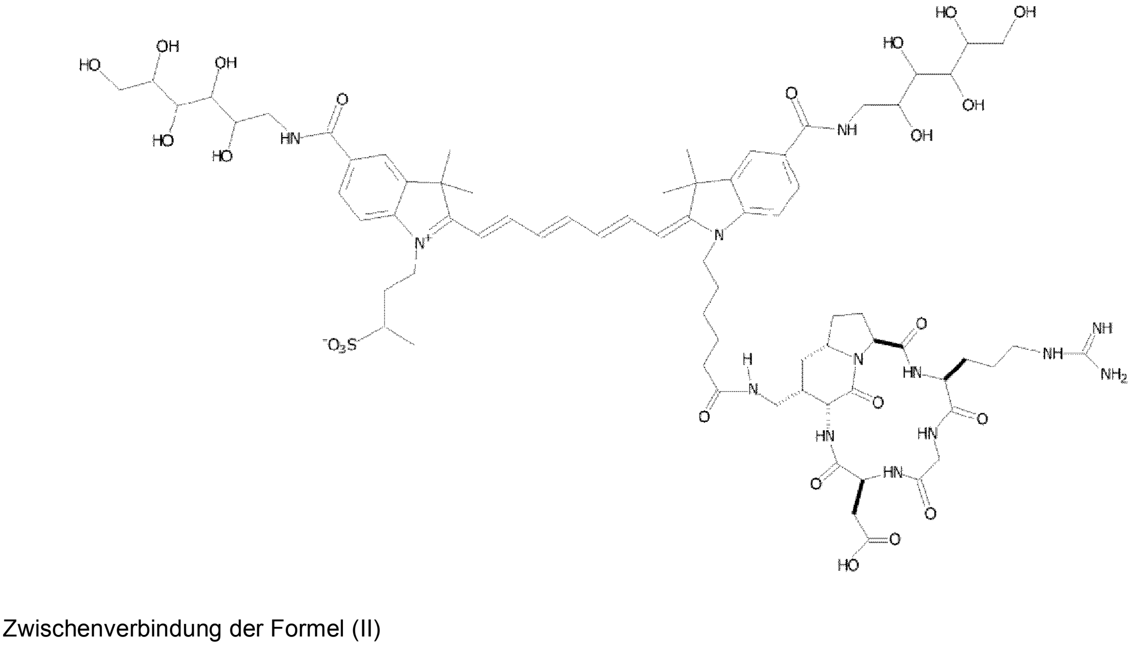

**13.** Zwischenverbindung der Formel (II)

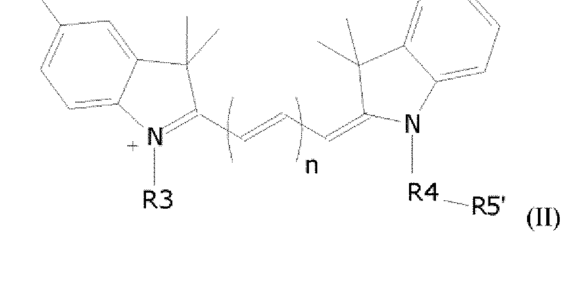

wobei

**R1, R2, R3, R4** und **n** wie in Anspruch 1 definiert sind und
**R5'** aus -COOH, $-CONH_2$, -COO-Pg und -CONH-Pg ausgewählt ist, wobei Pg für eine Schutzgruppe steht, oder ein pharmazeutisch unbedenkliches Salz davon.

**14.** Zwischenprodukt der Formel (II) nach Anspruch 13, wobei n für 3 steht, R3 für Alkyl, das durch $-SO_3H$ substituiert ist, steht und R1 und R2 unabhängig aus der Gruppe bestehend aus

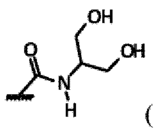 (iii), 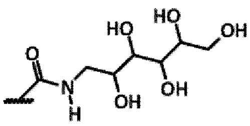 (iv), 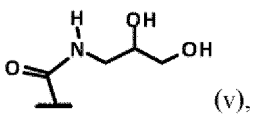 (v), 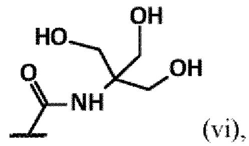 (vi),

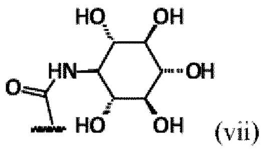 (vii) und 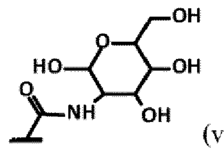 (viii)

ausgewählt sind.

**15.** Zwischenprodukt der Formel (II) nach Anspruch 13 oder 14, die durch Formel (IIa) wiedergegeben wird:

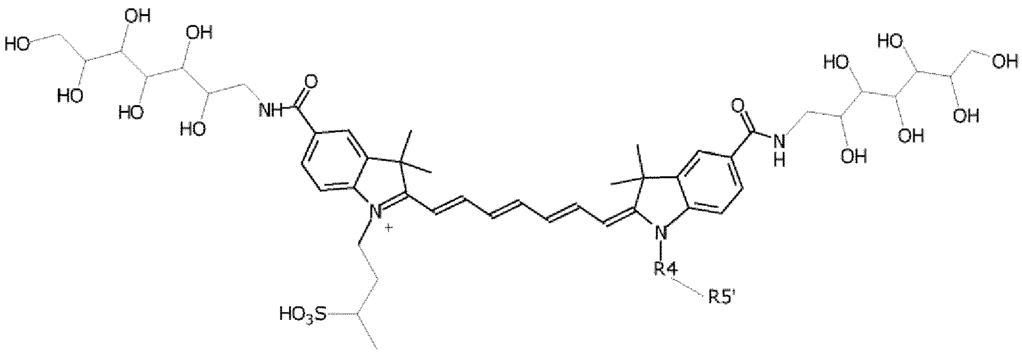

(IIa)

wobei R4 und R5' wie in Anspruch 13 definiert sind.

## Revendications

**1.** Composé de formule (I)

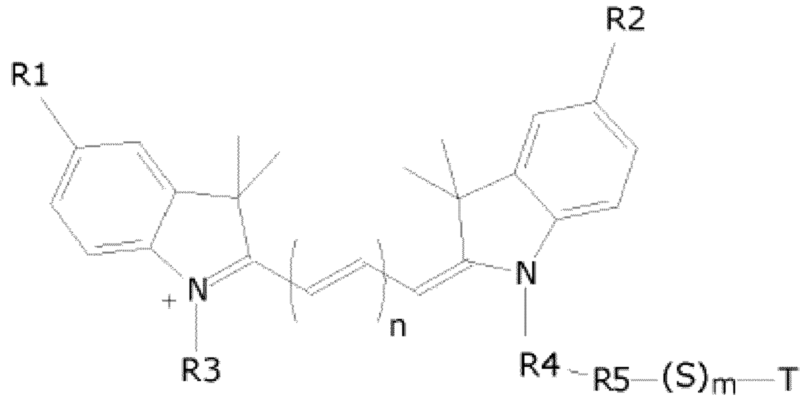

(I)

R1 et R2 étant indépendamment un groupe -CO-NH-Y, Y étant choisi parmi un alkyle linéaire ou ramifié, un cycloalkyle et un hétérocyclyle, substitué par au moins deux groupes hydroxyle ;
R3 étant alkyle linéaire ou ramifié substitué par un groupe choisi parmi $-NH_2$, $-SO_3H$, -COOH et $-CONH_2$ ; R4 étant alkyle bivalent linéaire ou ramifié ;
R5 étant choisi parmi -COO- et -CONH- ;
S étant un espaceur ;
T étant un groupement de ciblage ;
η étant un entier égal à 1, 2 ou 3 ; et
m étant un entier égal à 0 ou 1,
ou sel pharmaceutiquement acceptable correspondant.

2. Composé de formule (I) selon la revendication 1, n étant 3, R3 étant alkyle substitué par $-SO_3H$, et R1 et R2 étant indépendamment choisis dans le groupe constitué par

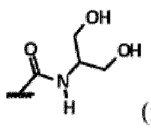 (iii), 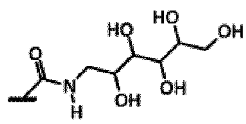 (iv), 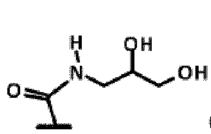 (v), 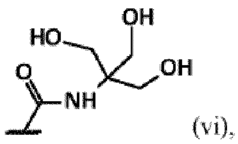 (vi),

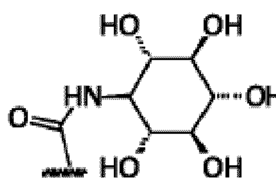 (vii) et 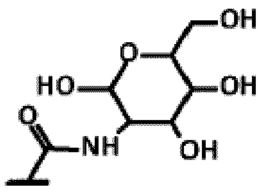 (viii).

3. Composé de formule (I) selon la revendication 1 ou 2, qui est représenté par la formule (Ia)

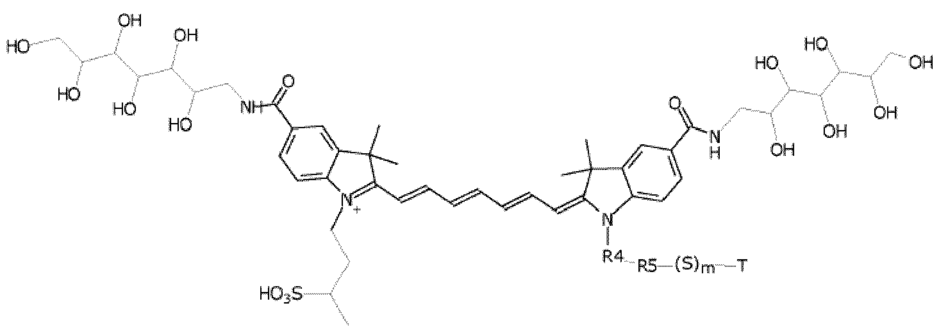

(Ia) ,

R4, R5, S, m et T étant tels que définis dans la revendication 1.

4. Composé de formule (I) selon la revendication 1, 2 ou 3, S étant choisi parmi $-NH(CH_2)_PCOO-$,$-NH(CH_2CH_2O)_pCH_2CH_2COO-$ et $-NH(CH_2CH_2O)_pCH_2CH_2NH-$, p étant un entier compris entre 0 et 20.

5. Composé de formule (I) selon l'une quelconque des revendications précédentes, T étant un groupement de ciblage choisi dans le groupe constitué par une petite molécule, une protéine, un peptide, un peptidomimétique, un substrat d'enzyme, un anticorps ou un fragment correspondant et un aptamère.

6. Composé de formule (I) selon l'une quelconque des revendications précédentes, T étant un groupement interagissant avec un récepteur d'intégrines.

7. Composé de formule (I) selon l'une quelconque des revendications précédentes pour une utilisation en tant qu'agent de contraste de fluorescence pour la détection et la délimitation d'une marge tumorale dans une chirurgie guidée d'un patient individuel.

8. Composé de formule (I) pour une utilisation selon la revendication 7, la détection et la délimitation de la marge tumorale étant réalisée sous irradiation NIR.

9. Composé de formule (I) pour une utilisation selon la revendication 8, ladite tumeur étant une tumeur choisie parmi un cancer du cerveau, un cancer du sein, un cancer de la tête et du cou, un cancer de l'ovaire, un cancer de la prostate, un cancer de l'œsophage, un cancer de la peau, un cancer gastrique, un cancer pancréatique, un cancer de la vessie, un cancer oral, un cancer du poumon, un cancer rénal, un cancer utérin, un cancer de la thyroïde, un cancer du foie, et un cancer colorectal.

10. Composition de diagnostic pharmaceutique comprenant un composé de formule (I) tel que défini dans la revendication 1 et au moins un support ou excipient pharmaceutiquement acceptable.

11. Kit de diagnostic comprenant un composé de formule (I) tel que défini dans la revendication 1 conjointement avec des adjuvants supplémentaires de celui-ci pour implémenter l'imagerie optique.

12. Composé de formule (I) selon la revendication 1 choisi parmi

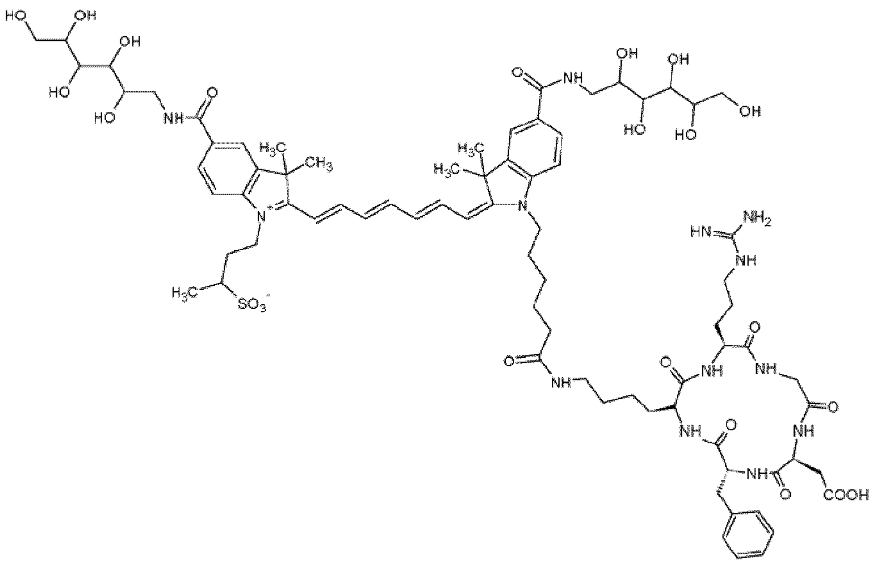

et

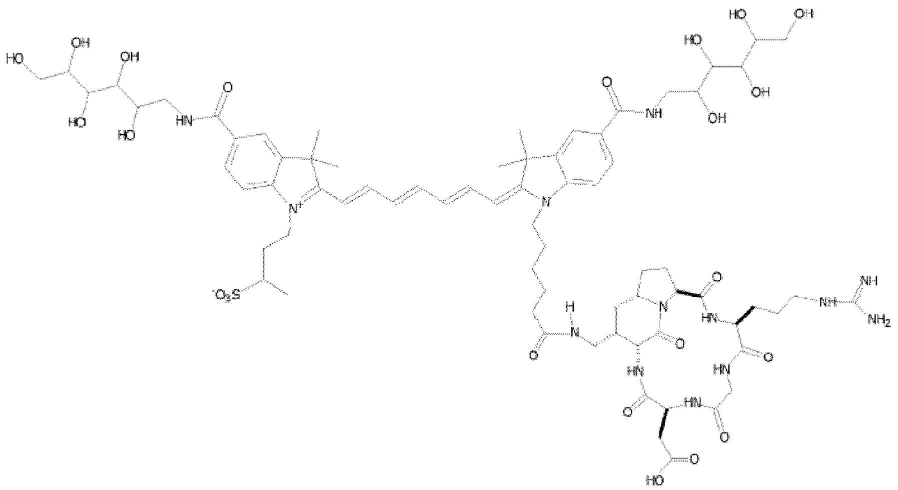

.

**13.** Composé intermédiaire de formule (II)

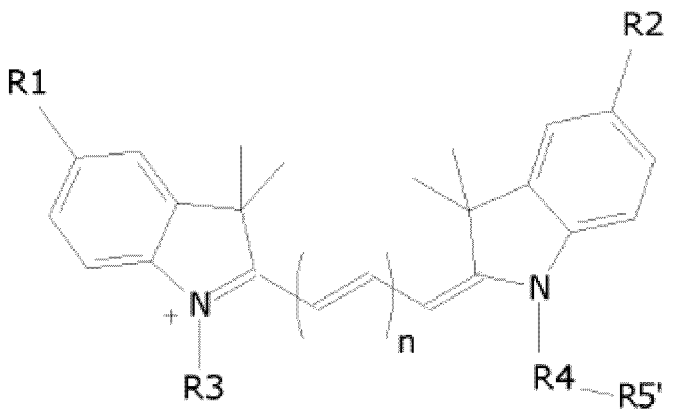

(II)

R1, R2, R3, R4 et n étant tels que définis dans la revendication 1 et R5' étant choisi parmi -COOH, $-CONH_2$, $-COO-P_g$ et$-CONH-P_g$, $P_g$ étant un groupe protecteur, ou sel pharmaceutiquement acceptable correspondant.

**14.** Intermédiaire de formule (II) selon la revendication 13, n étant 3, R3 étant alkyle substitué par $-SO_3H$, et R1 et R2

étant indépendamment choisis dans le groupe constitué par

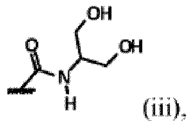 (iii), 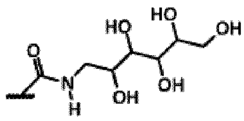 (iv), 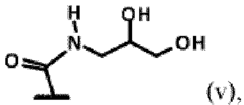 (v), 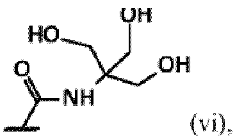 (vi),

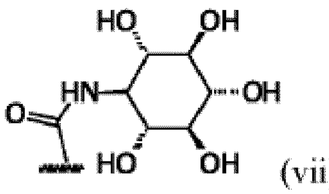 (vii) et 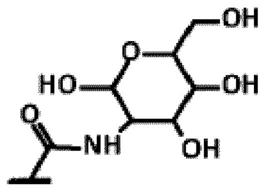 (viii).

**15.** Intermédiaire de formule (II) selon la revendication 13 ou 14, qui est représenté par la formule (IIa)

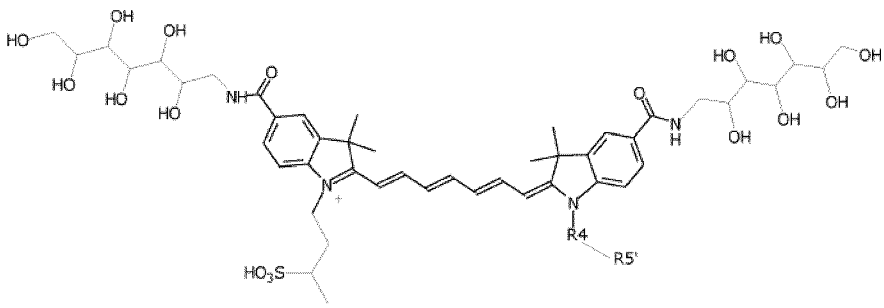

(IIa) ,

R4 et R5' étant tels que définis dans la revendication 13.

Figure 1

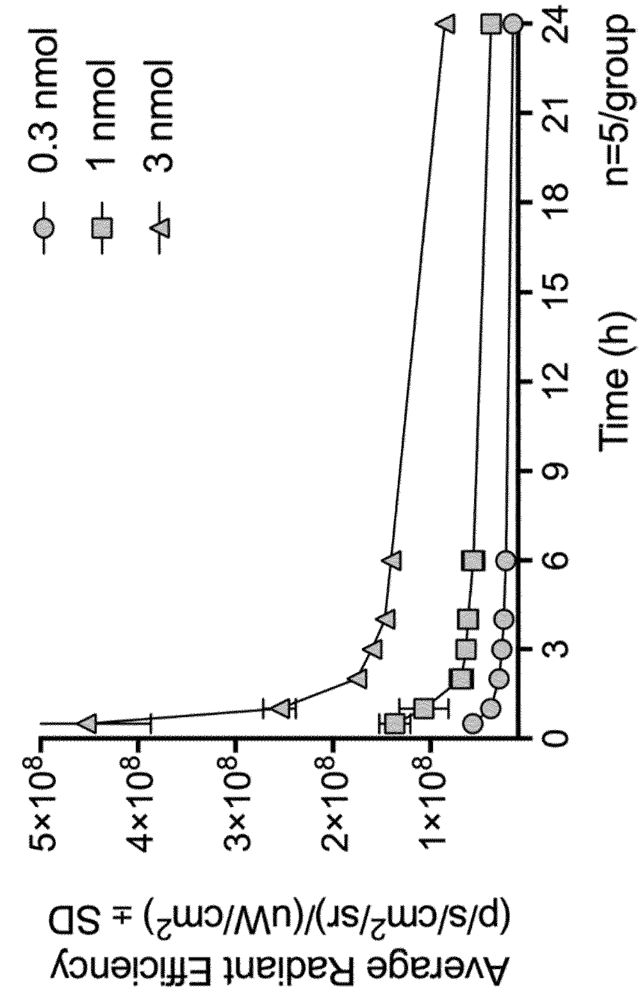
Average Radiant Efficiency
(p/s/cm²/sr)/(uW/cm²) ± SD
5×10⁸
4×10⁸
3×10⁸
2×10⁸
1×10⁸
0
3
6
9
12
15
18
21
24
Time (h)
n=5/group
0.3 nmol
1 nmol
3 nmol

Figure 2

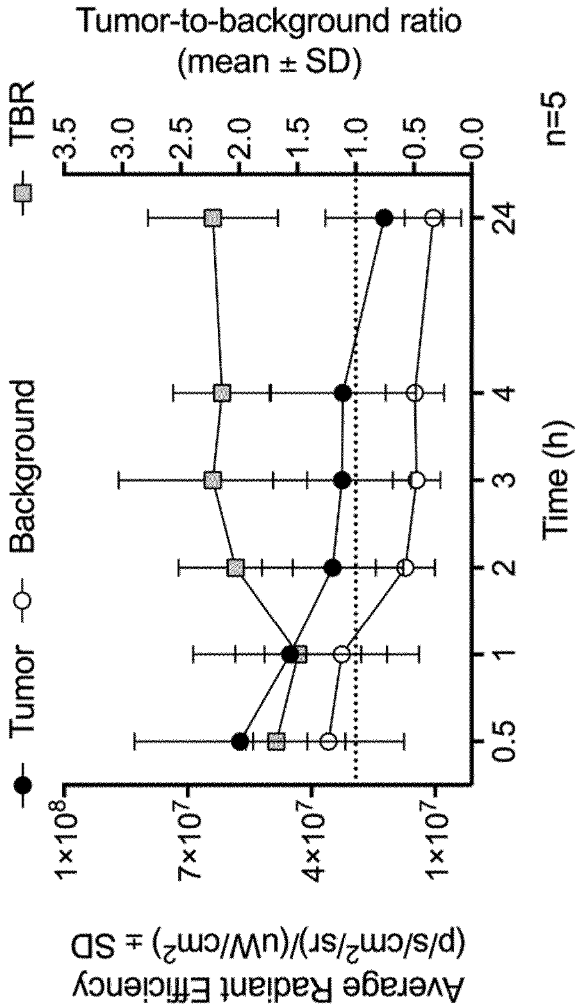
Tumor
Background
TBR
Average Radiant Efficiency
(p/s/cm²/sr)/(uW/cm²) ± SD
Tumor-to-background ratio
(mean ± SD)
Time (h)
n=5
1×10^8
7×10^7
4×10^7
1×10^7
3.5
3.0
2.5
2.0
1.5
1.0
0.5
0.0
0.5
1
2
3
4
24

Figure 3

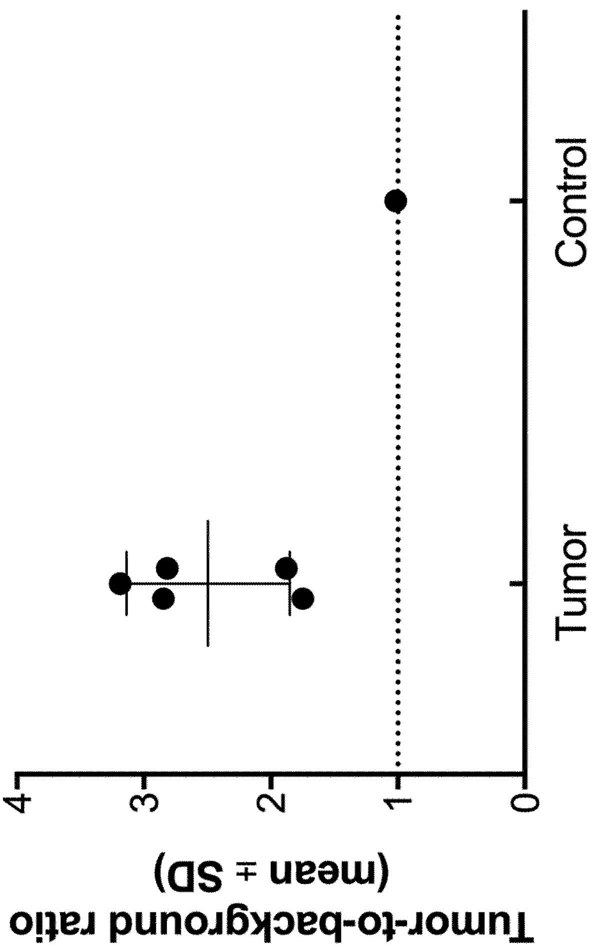

Figure 4

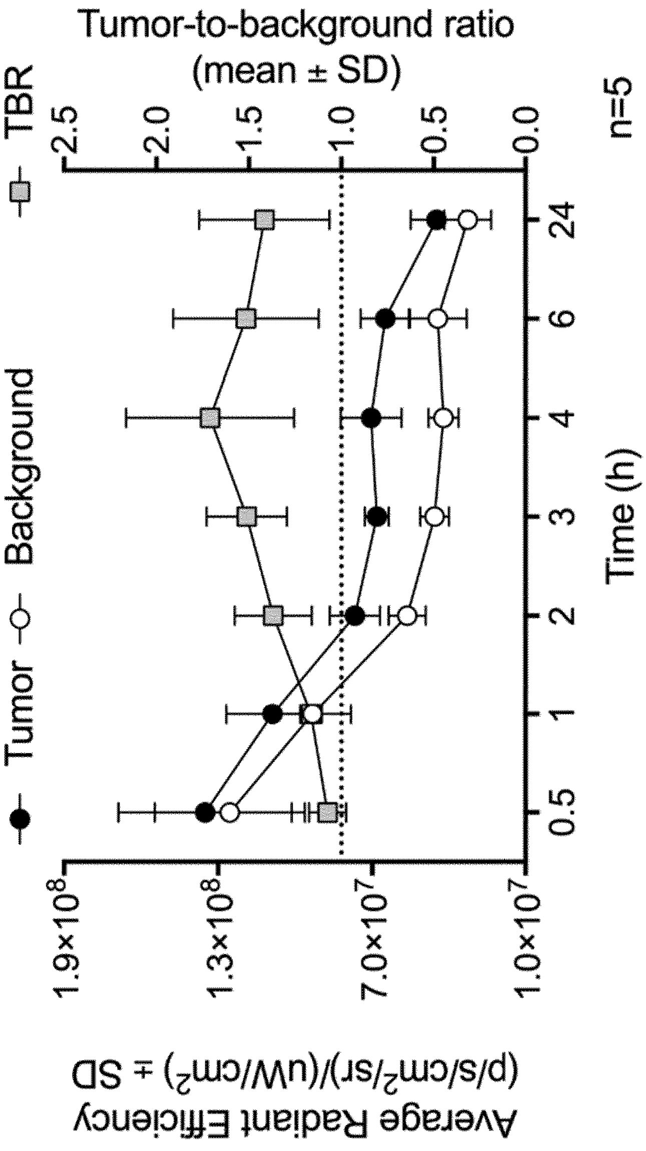
Tumor
Background
TBR
Tumor-to-background ratio
(mean ± SD)
2.5
2.0
1.5
1.0
0.5
0.0
n=5
Average Radiant Efficiency
(p/s/cm²/sr)/(uW/cm²) ± SD
1.9×10⁸
1.3×10⁸
7.0×10⁷
1.0×10⁷
0.5
1
2
3
4
6
24
Time (h)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6913743 B2
- WO 2018189136 A
- WO 2016097317 A
- US 6329531 B1
- WO 2005123768 A
- WO 2012088007 A


### Non-patent literature cited in the description


- **ONDA N. et al.** *Int J Cancer,* 2016, vol. 139, 673-682
- **STUMMER W. et al.** *Neurosurgery,* 2015, vol. 77 (5), 663-673
- **LICHA et al.** *Photochemistry and Photobiology,* 2000, vol. 72 (3), 392-398
- **TUMMERS Q. et al.** *PlosOne,* 2015
- **ACHILEFU S. et al.** *J Med Chem,* 2002, vol. 45, 2003-2015
- **BUNSCHOTEN A. et al.** *Bioconjugate Chem.,* 2016, vol. 27, 1253-1258
- **CAPOZZA et al.** *Photoacoustics,* 2018, vol. 11 (3), 6-45
- **EBERT et al.** *J. Biomed. Optics,* 2011, vol. 16 (6), 066003
- **T.W. GREEN ; P.G.M.WUTS.** Protective Groups in Organic Synthesis. Wiley, 2007
- **KAPP et al.** *Sci Rep,* 2017, vol. 7, 3905
- **WICHERT et al.** *Nat Chem,* 2015, vol. 7, 241-249
- **LI et al.** *FASEB J,* 2005, vol. 19, 1978-85
- **WILLIAMS et al.** *Chem Biol Drug Des,* 2018, vol. 91, 605-619
- **MUJUMDAR R.B. et al.** *Bioconjugate Chem.,* 1993, vol. 4 (2), 105-111
- **YAMANA et al.** *Chem. Pharm. Bull.,* 1972, vol. 20 (5), 881-891
- *CHEMICAL ABSTRACTS,* 339177-26-3
- *CHEMICAL ABSTRACTS,* 205923-56-4
- **KAPP et al.** *Sci. Rep.,* 2017, vol. 7, 39805
- **CAPASSO et al.** *PlosOne,* 2014
- **CAPOZZA et al.** *Photoacoustic,* 2018, vol. 11, 36-45
- **CAPOZZA et al.** *Photoacoustics,* 2018, vol. 11, 36-45